# EUROPEAN PATENT SPECIFICATION

(11) **EP 3 954 677 B1**
(45) Date of publication and mention of the grant of the patent: **23.10.2024**
(21) Application number: 20844060.2
(22) Date of filing: 20.07.2020
(51) Int. Cl.: C07C 211/61, C09K 11/06

(54) **NOVEL COMPOUND AND ORGANIC LIGHT EMITTING DEVICE COMPRISING SAME**
NEUARTIGE VERBINDUNG UND DIESE ENTHALTENDE ORGANISCHE LICHTEMITTIERENDE VORRICHTUNG
NOUVEAU COMPOSÉ ET DISPOSITIF ÉLECTROLUMINESCENT ORGANIQUE LE COMPRENANT

(30) Priority: 24.07.2019 KR 20190089701
(43) Date of publication of application: 16.02.2022
(73) Proprietor: LG Chem, Ltd., Seoul 07336 (KR)
(72) Inventor: LEE, Jiyoung, Daejeon 34122 (KR); BAE, Jaesoon, Daejeon 34122 (KR); LEE, Jaechol, Daejeon 34122 (KR); CHOI, Doowhan, Daejeon 34122 (KR); KANG, Sungkyoung, Daejeon 34122 (KR); SHIN, Hyeonah, Daejeon 34122 (KR); YI, Yeonhui, Daejeon 34122 (KR); JUNG, Min Suk, Daejeon 34122 (KR)
(74) Representative: Hoffmann Eitle
(86) International application number: PCT/KR2020/009556
(87) International publication number: WO 2021/015522

(56) References cited:
- WO-A1-2017/100967
- KR-A- 20140 107 594
- KR-A- 20140 132 562
- KR-A- 20150 093 995
- KR-A- 20180 092 270
- KR-A- 20180 092 270

## Description

### TECHNICAL FIELD

The present disclosure relates to a novel compound and an organic light emitting device comprising the same.

### BACKGROUND ART

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for - the organic materials used in the organic light emitting devices as described above.

Meanwhile, recently, in order to reduce process costs, an organic light emitting device using a solution process, particularly an inkjet process, has been developed instead of a conventional deposition process. In the initial stage of development, attempts have been made to develop organic light emitting devices by coating all organic light emitting device layers by a solution process, but current technology has limitations. Therefore, only HIL, HTL, and EML are processed in a layer device structure by a solution process, and a hybrid process utilizing traditional deposition processes is being studied as a subsequent process.

In this regard, the present disclosure provides novel materials for organic light emitting devices that can be used for an organic light emitting device and simultaneously, can be deposited by a solution process.

### Prior Art Literature

### Patent Literature

Patent Literature 1: Korean Unexamined Patent Publication No. 10-2000-0051826
KR 10-2018-0092270 A discloses compounds for inclusion in an organic light emitting device.

### DETAILED DESCRIPTION OF THE INVENTION

### Technical Problem

It is an object of the present disclosure to provide a novel compound and an organic light emitting device comprising the same.

### Technical Solution

According to an aspect of the present disclosure, there is provided a compound represented by the following Chemical Formula 1: 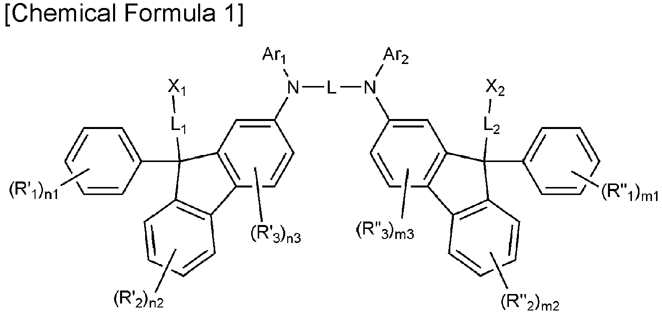 in Chemical Formula 1,
L is substituted or unsubstituted C₆₋₆₀ arylene; or substituted or unsubstituted C₂₋₆₀ heteroarylene containing any one or more heteroatoms selected from N, O and S;
L₁ and L₂ are each independently a single bond or methylene;
X₁ and X₂ are each independently a photocurable group or a thermosetting group independently represented by -L"-R", wherein L" is a single bond, -O-, -S-, -CH₂-, -CH₂O-, -OCH₂-, or -CH₂OCH₂-, and R" is any one of the following: 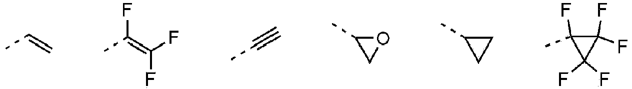 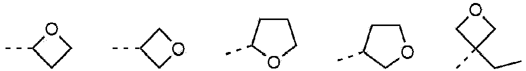 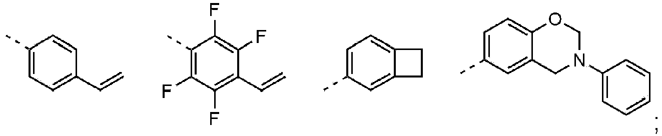
R'₁ to R'₃ and R"₁ to R"3 are each independently hydrogen, deuterium, substituted or unsubstituted C₁₋₆₀ alkyl, substituted or unsubstituted C₁₋₆₀ alkoxy, substituted or unsubstituted C₆₋₆₀ aryl, or a substituted or unsubstituted C₂₋₆₀ heteroaryl containing any one or more heteroatoms selected from N, O and S;
n1 to n3 and m1 to m3 are each independently an integer of 0 to 3; and
Ar₁ and Ar₂ are each independently a substituent group represented by the following Chemical Formula 2: 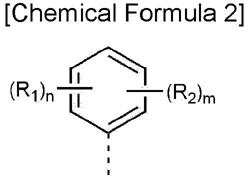 in Chemical Formula 2,
   each R₁ is independently a halogen;
   each R₂ is independently hydrogen, deuterium, or C₁₋₁₀ alkyl; and
   n is an integer of 1 to 5, and m is 0 or 1, provided that n+m is 5 or less.

According to another aspect of the present disclosure, there is provided an organic light emitting device comprising a first electrode; a second electrode provided opposite the first electrode; and one or more organic material layers provided between the first electrode and the second electrode, wherein one or more of the organic material layers include a cured product of the above-mentioned compound.

### ADVANTAGEOUS EFFECTS

The above-mentioned compound represented by Chemical Formula 1 can be used as a material of an organic material layer of an organic light emitting device, can be used for a solution process, and can improve the efficiency, achieve low driving voltage and/or improve lifetime characteristics in the organic light emitting device.

### BRIEF DESCRIPTION OF THE DRAWINGS

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 7, an electron injection and transport layer 8, and a cathode 4.

FIGS. 3 to 7 show NMR data of each compound prepared in Examples of the present disclosure.

### DETAILED DESCRIPTION OF THE EMBODIMENTS

Hereinafter, embodiments of the present disclosure will be described in more detail to facilitate understanding of the invention.

### Definition of Terms

As used herein, the notation 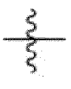 or 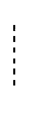 means a bond linked to another substituent group.

As used herein, the term "substituted or unsubstituted" means being unsubstituted or substituted with one or more substituents selected from the group consisting of deuterium; a halogen group; a cyano group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl group containing at least one of N, O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected.

In the present disclosure, the carbon number of a carbonyl group is not particularly limited, but is preferably 1 to 40. Specifically, the carbonyl group may be a compound having the following structural formulas, but is not limited thereto.

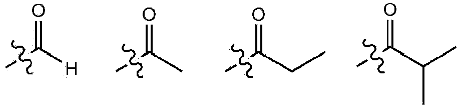

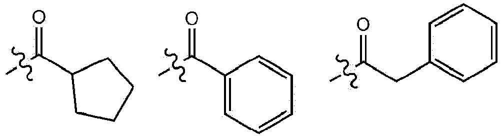

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a compound having the following structural formulas, but is not limited thereto.

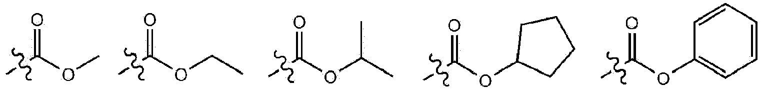

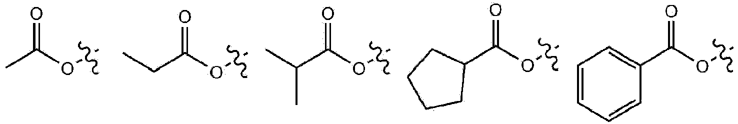

In the present disclosure, the carbon number of an imide group is not particularly limited, but is preferably 1 to 25. Specifically, the imide group may be a compound having the following structural formulas, but is not limited thereto.

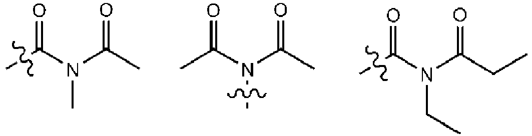

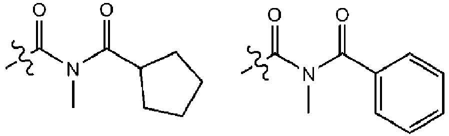

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto.

In the present disclosure, the fluorenyl group may be substituted, and two substituents may be linked with each other to form a spiro structure. In the case where the fluorenyl group is substituted, 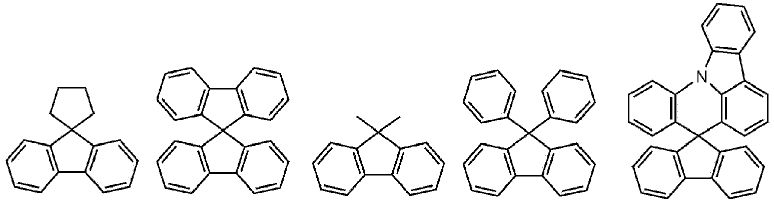 and the like can be formed. However, the structure is not limited thereto.

In the present disclosure, a heteroaryl group is a heteroaryl group containing one or more of O, N, Si and S as a heteroatom, and the carbon number thereof is not particularly limited, but is preferably 2 to 60. Examples of the heteroaryl group include a xanthene group, a thioxanthene group, a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, the arylamine group and the arylsily group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can be applied to the aforementioned description of the heteroaryl group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heteroaryl group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heteroaryl group is not a monovalent group but formed by combining two substituent groups.

### Compound

The present disclosure provides a compound represented by Chemical Formula 1 above.

Preferably, L is phenylene, biphenyldiyl, or spirobifluorenediyl. More preferably, L is any one selected from the group consisting of:

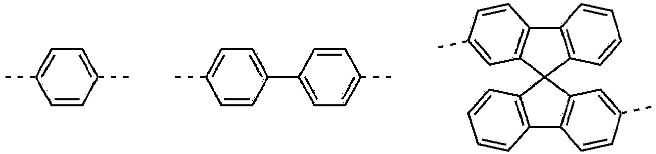

Preferably, L₁ and L₂ are single bonds.

Preferably, R₁ is fluoro.

Preferably, R₂ is hydrogen; deuterium; or methyl.

Preferably, Ar₁ and Ar₂ are each independently a substituent group represented by: 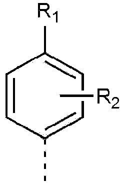 wherein,
R₁ is C₁₋₁₀ alkyl; or a halogen,
R₂ is hydrogen; deuterium; C₁₋₁₀ alkyl; or a halogen,
with the proviso that at least one of R₁ and R₂ are a halogen.

Preferably, Ar₁ and Ar₂ are each independently any one selected from the group consisting of:

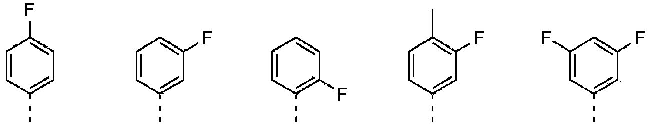

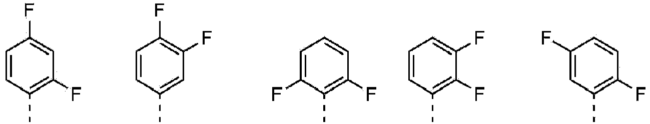

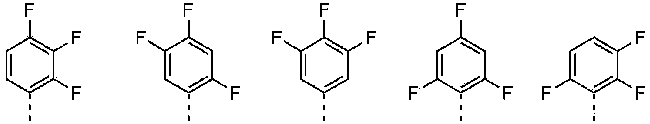

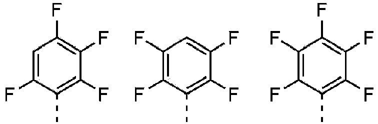

Preferably, Ar₁ and Ar₂ are the same as each other.

X₁ and X₂ are each independently -L"-R", L" is a single bond, -O-, -S-, -CH₂-, -CH₂O-, -OCH₂-, or -CH₂OCH₂-, and R" is any one selected from the group consisting of:

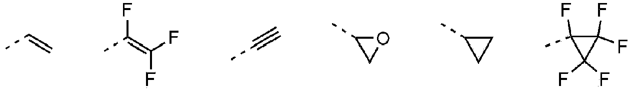

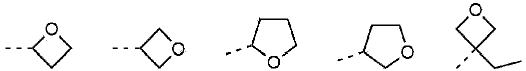

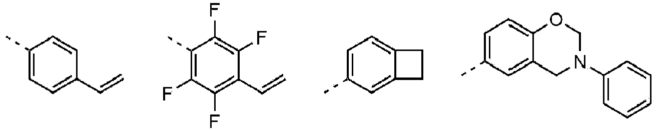

Preferably, R'₁ and R"₁ are each independently hydrogen or methyl, and n1 and m1 are each independently an integer of 0 to 2. Also preferably, R'₁ and R"₁ are the same as each other.

Preferably, R'₂, R'₃, R"₂ and R"₃ are hydrogen.

Representative examples of the compound represented by Chemical Formula 1 are as follows:

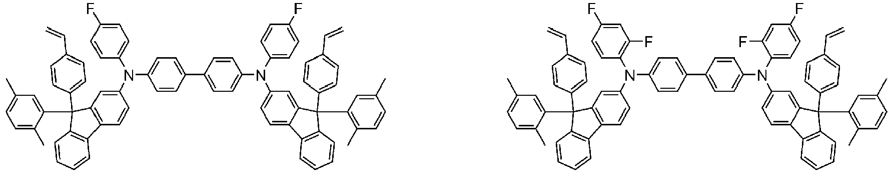

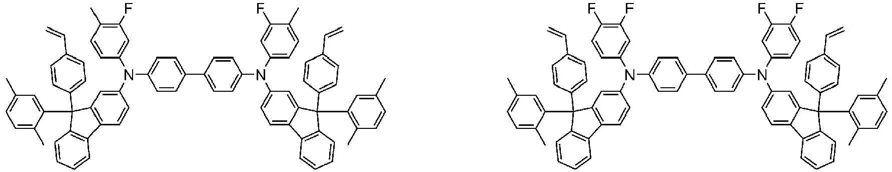

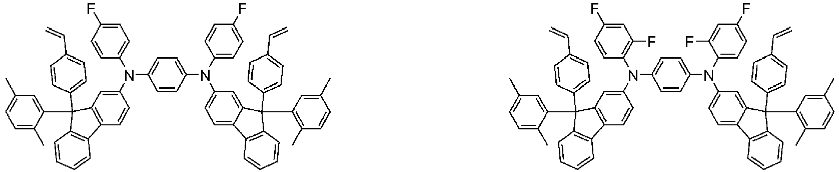

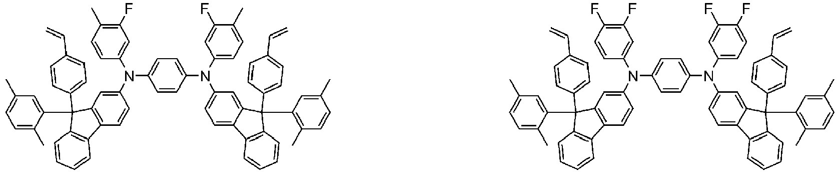

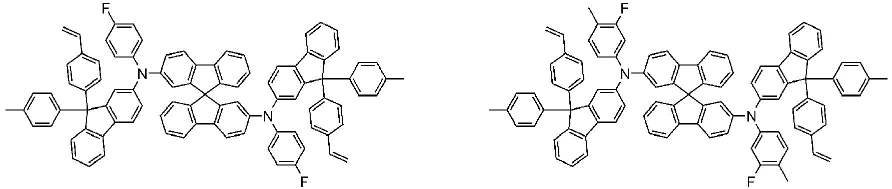

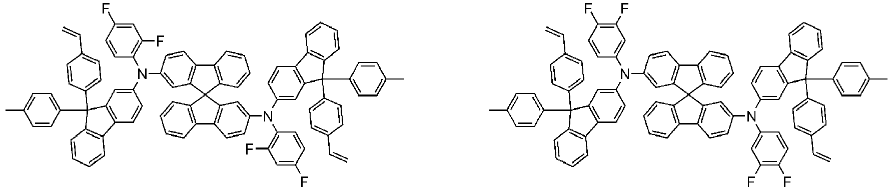

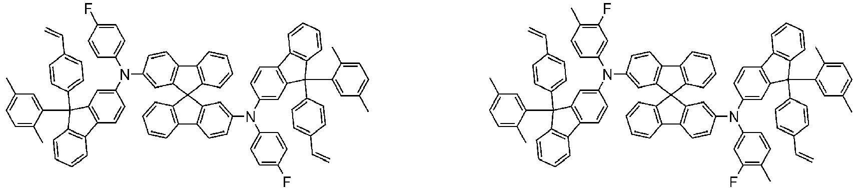

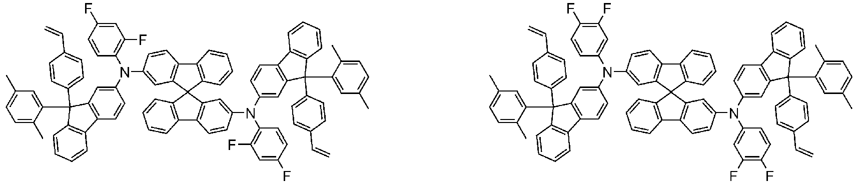

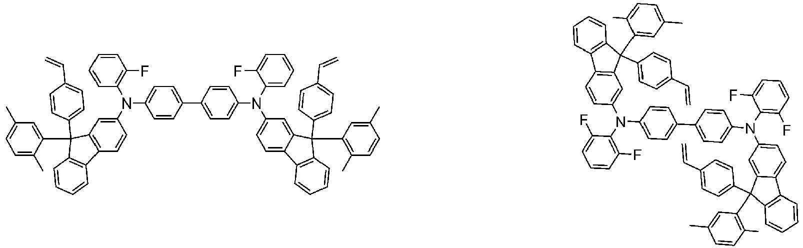

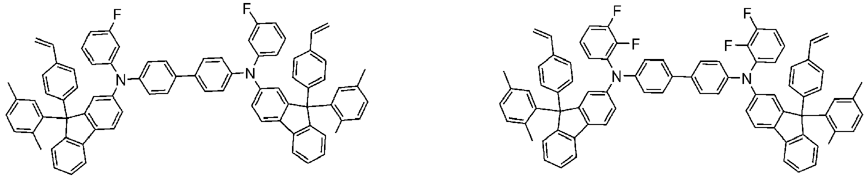

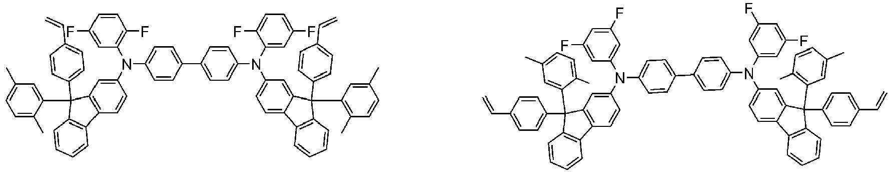

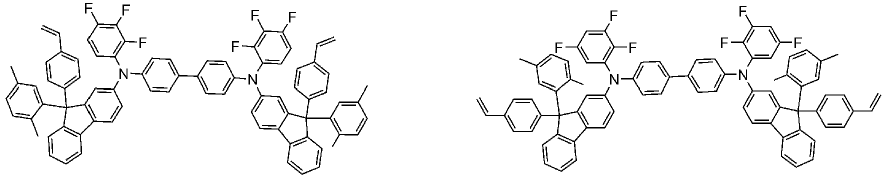

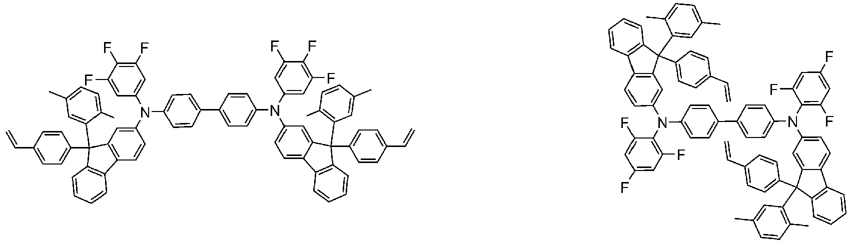

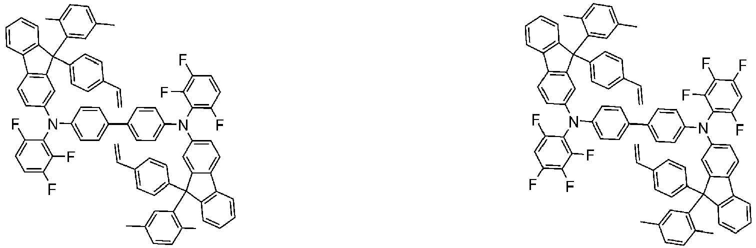

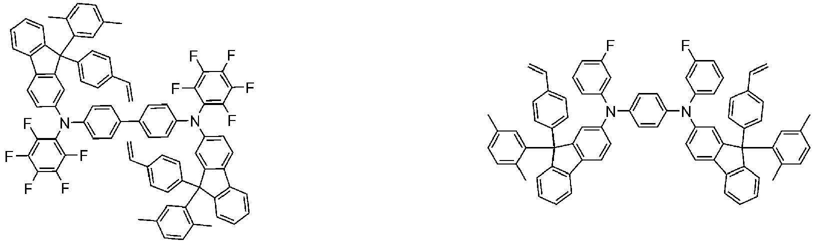

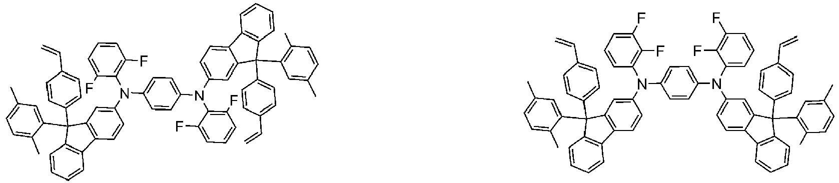

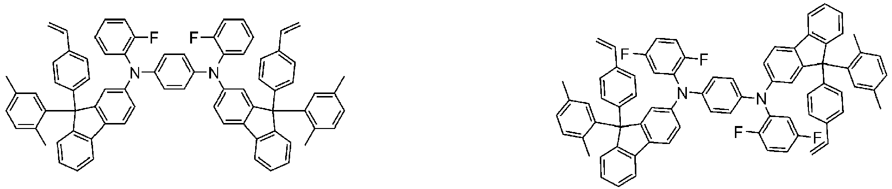

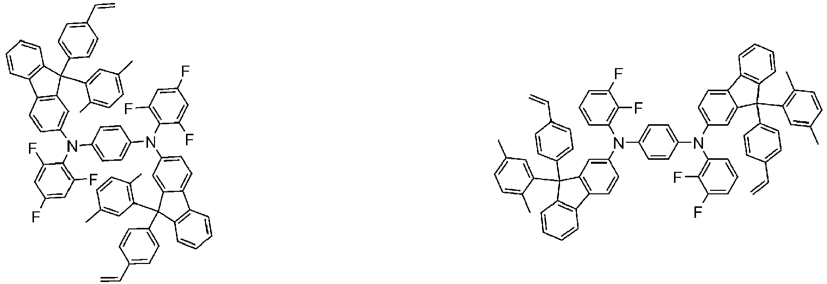

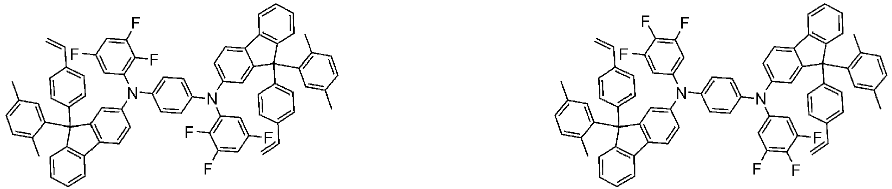

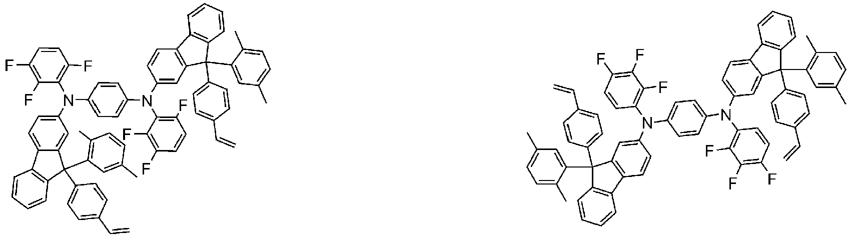

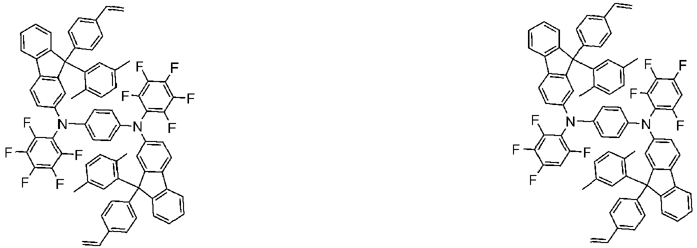

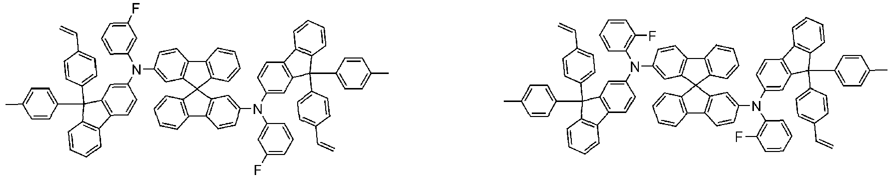

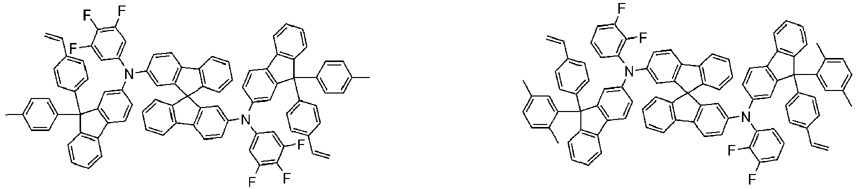

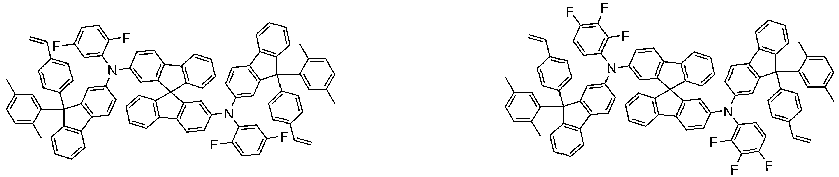

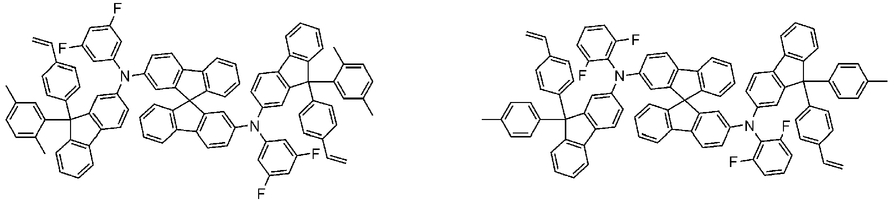

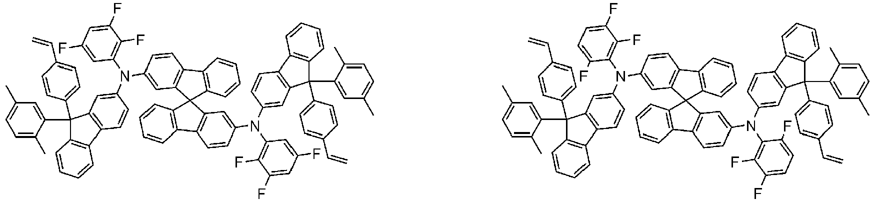

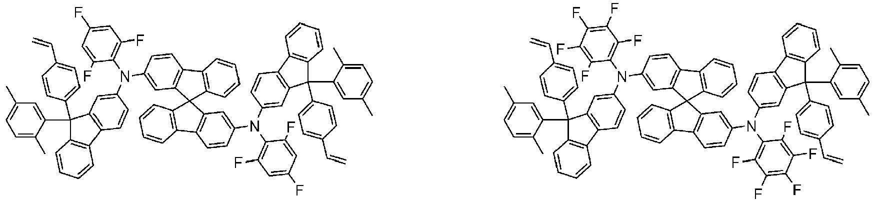

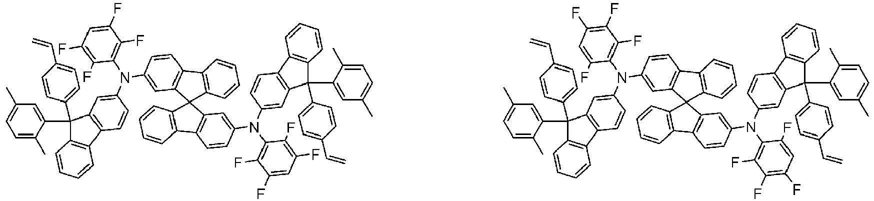

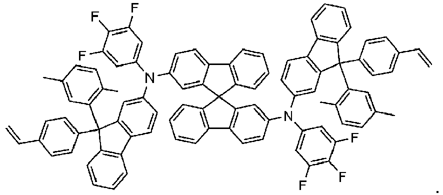

A method for preparing the compound represented by Chemical Formula 1 is shown in the following Reaction Scheme 1.

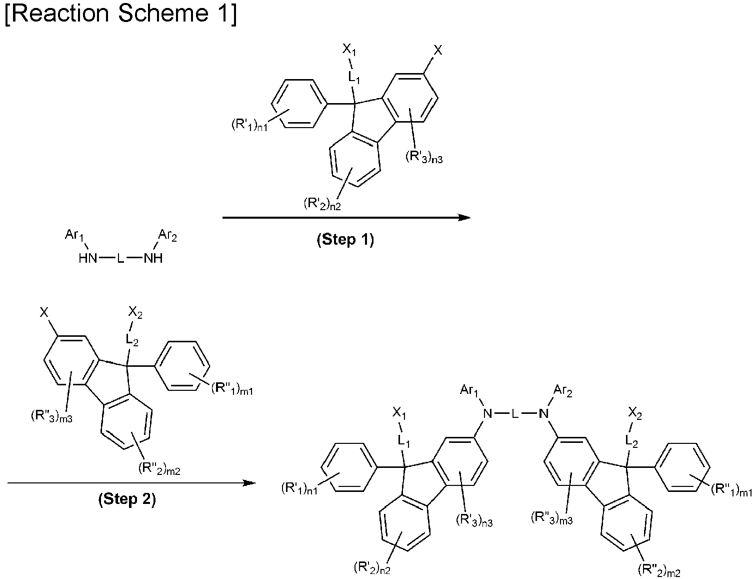

In the Reaction Scheme 1, the definition of the remaining substituent group except for X are the same as defined above, and X is halogen, and more preferably, chloro or bromo.

If the final compound in Reaction Scheme 1 has a bilaterally symmetrical structure, step 2 can be omitted. The reaction of step 1 and step 2 above is an amine substitution reaction which is preferably carried out in the presence of a palladium catalyst and a base, and a reactive group for the amine substitution reaction can be modified as known in the art. The above preparation method may be further embodied in the Preparation Examples described hereinafter.

### Coating composition

The compound according to the present disclosure can form an organic material layer, particularly a hole transport layer, of an organic light emitting device by a solution process. For this purpose, a coating composition can be prepared comprising the above-mentioned compound according to the present disclosure and a solvent.

The solvent is not particularly limited as long as it is a solvent capable of dissolving or dispersing the compound according to the present disclosure. Examples of the solvent may include chlorine-based solvents such as chloroform, methylene chloride, 1,2-dichloroethane, 1,1,2-trichloroethane, chlorobenzene and o-dichlorobenzene; ether-based solvents such as tetrahydrofuran and dioxane; aromatic hydrocarbon-based solvents such as toluene, xylene, trimethylbenzene and mesitylene; aliphatic hydrocarbon-based solvents such as cyclohexane, methylcyclohexane, n-pentane, n-hexane, n-heptane, n-octane, n-nonane and n-decane; ketone-based solvents such as acetone, methyl ethyl ketone, and cyclohexanone; ester-based solvents such as ethyl acetate, butyl acetate and ethyl cellosolve acetate; polyalcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin and 1,2-hexanediol, and derivatives thereof; alcohol-based solvents such as methanol, ethanol, propanol, isopropanol and cyclohexanol; sulfoxide-based solvents such as dimethyl sulfoxide; amide-based solvents such as N-methyl-2-pyrrolidone and N,N-dimethylformamide; benzoate-based solvents such as butyl benzoate and methyl-2-methoxybenzoate; tetraline; 3-phenoxy-toluene, and the like. In addition, the above-mentioned solvents may be used singly or in combination of two or more solvents.

Further, the viscosity of the coating composition is preferably 1 cP to 10 cP, and coating is easy within the above range. Further, in the coating composition, the concentration of the compound according to the present disclosure is preferably 0.1 wt/v% to 20 wt/v%.

Further, the coating composition may further include one, two or more types of additives selected from the group consisting of a thermal polymerization initiator and a photopolymerization initiator.

Examples of the thermal polymerization initiator may include peroxides such as methyl ethyl ketone peroxide, methyl isobutyl ketone peroxide, acetyl acetone peroxide, methyl cyclohexanone peroxide, cyclohexanone peroxide, isobutyryl peroxide, 2,4-dichlorobenzoyl peroxide, bis-3,5,5-trimethylhexanoyl peroxide, lauryl peroxide, benzoyl peroxide, or azo-based such as azobis isobutylnitrile, azobis dimethylvaleronitrile and azobis cyclohexylnitrile, but are not limited thereto.

Examples of the photopolymerization initiator may include acetophenone-based or ketal-based photopolymerization initiators such as diethoxyacetophenone, 2,2-dimethoxy-1,2-diphenylethan-1-one, 1-hydroxycyclohexyl-phenyl-ketone, 4-(2-hydroxyethoxy)phenyl-(2-hydroxy-2-propyl)ketone, 2-benzyl-2-dimethylamino-1 -(4-morpholinophenyl)butanone-1,2-hydroxy-2-methyl-1 -phenylpropan-1 -one, 2-methyl-2-morpholino(4-methylthiophenyl)propan-1-one and 1-phenyl-1,2-propanedion-2-(o-ethoxycarbonyl)oxime, benzoin ether-based photopolymerization initiators such as benzoin, benzoin methyl ether, benzoin ethyl ether, benzoin isobutyl ether and benzoin isopropyl ether, benzophenone-based photopolymerization initiators such as benzophenone, 4-hydroxybenzophenone, 2-benzoyl naphthalene, 4-benzoylbiphenyl and 4-benzoylphenyl ether, thioxanthone-based photopolymerization initiators such as 2-isopropylthioxanthone, 2-chlorothioxanthone, 2,4-dimethylthioxanthone, 2,4-diethylthioxanthone and 2,4-dichlorothioxanthone, and other photopolymerization initiators such as ethyl anthraquinone, 2,4,6-trimethylbenzoyldiphenylphosphine oxide, 2,4,6-trimethylbenzoylphenylethoxyphosphine oxide, bis(2,4,6-trimethylbenzoyl)phenylphosphine oxide, bis(2,4-dimethoxy benzoyl)-2,4,4-trimethylpentylphosphine oxide, but are not limited thereto.

Moreover, those having a photopolymerization promoting effect can also be used alone or in combination with the photopolymerization initiator. Examples thereof include triethanolamine, methyldiethanolamine, ethyl 4-dimethylaminobenzoate, isoamyl 4-dimethylamino benzoate, (2-dimethylamino)ethyl benzoate, 4,4'-dimethylaminobenzophenone, and the like, but are not limited thereto.

A hole transport layer can be produced using the above-mentioned coating composition. Specifically, the method can include the steps of coating the above-mentioned coating composition onto the anode or onto the hole injection layer formed on the anode by a solution process, and heat-treating the coated coating composition.

The solution process uses the coating composition and refers to spin coating, dip coating, doctor blading, inkjet printing, screen printing, spray method, roll coating, and the like, but is not limited thereto.

The heat treatment temperature in the heat treatment step is preferably from 150 to 230°C. A heat treatment time may be from 1 minute to 3 hours, more preferably 10 minutes to 1 hour. In another example, the heat treatment is preferably carried out in an inert gas atmosphere such as argon and nitrogen. Further, a step of evaporating a solvent may be further included between the coating step and the heat treatment or light treatment step.

### Organic Light Emitting Device

According to yet another embodiment of the present disclosure, there is provided an organic light emitting device comprising a cured product of the compound represented by Chemical Formula 1.

The organic light emitting device comprises a first electrode; a second electrode that is provided opposite the first electrode; and one or more organic material layers that are provided between the first electrode and the second electrode, wherein one or more of the organic material layers include a cured product of the compound according to the present disclosure.

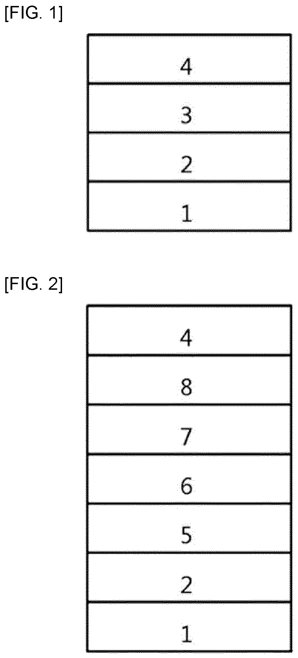

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of an organic light emitting device according to an embodiment of the present disclosure is illustrated in FIGS. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a light emitting layer 7, an electron injection and transport layer 8, and a cathode 4. In such a structure, the compound represented by Chemical Formula 1 may be included in the hole injection layer, the hole transport layer, or the light emitting layer.

The organic light emitting device according to the present disclosure may be manufactured by materials and methods known in the art, except that the light emitting layer includes the compound according to the present disclosure.

For example, the organic light emitting device according to the present disclosure can be manufactured by sequentially stacking an anode, an organic material layer and a cathode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon.

In addition to such a method, the organic light emitting device may be manufactured by sequentially depositing a cathode material, an organic material layer and an anode material on a substrate (International Publication WO2003/012890). However, the manufacturing method is not limited thereto.

As an example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive compounds such as poly(3-methylthiophene), poly[3,4-(ethylene-1,2-dioxy)thiophene] (PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive compound, and the like, but are not limited thereto.

The hole transport layer is a layer that receives holes from a hole injection layer and transports the holes to the light emitting layer. The hole transport layer is suitably a material having large mobility to the holes, which may receive holes from the anode or the hole injection layer and transfer the holes to the light emitting layer. Specific examples thereof include an arylamine-based organic material, a conductive compound, a block copolymer in which a conjugate portion and a non-conjugate portion are present together, and the like, but are not limited thereto.

The light emitting layer may include a host material and a dopant material. The host material may be a fused aromatic ring derivative, a heterocycle-containing compound or the like. Specific examples of the fused aromatic ring derivatives include anthracene derivatives, pyrene derivatives, naphthalene derivatives, pentacene derivatives, phenanthrene compounds, and fluoranthene compounds. Examples of the heterocycle-containing compound include carbazole derivatives, dibenzofuran derivatives, ladder-type furan compounds, pyrimidine derivatives, and the like, but are not limited thereto.

The dopant material includes an aromatic amine derivative, a styrylamine compound, a boron complex, a fluoranthene compound, a metal complex, or the like. Specifically, the aromatic amine derivative is a fused aromatic ring derivative having a substituted or unsubstituted arylamino group and includes arylamino group-including pyrene, anthracene, chrysene, peryflanthene and the like, and the styrylamine compound is a compound in which substituted or unsubstituted arylamine is substituted with at least one arylvinyl group, and one, two or more substituents selected from the group consisting of an aryl group, a silyl group, an alkyl group, a cycloalkyl group and an arylamino group are substituted or unsubstituted. Specifically, styrylamine, styryldiamine, styryltriamine, styryltetramine or the like is included, but the styrylamine compound is not limited thereto. In addition, the metal complex includes indium complexes, platinum complexes or the like, but is not limited thereto.

The electron transport layer is a layer which receives electrons from an electron injection layer and transports the electrons to a light emitting layer, and an electron transport material is suitably a material which may receive electrons well from a cathode and transfer the electrons to a light emitting layer, and has a large mobility for electrons. Specific examples of the electron transport material include: an Al complex of 8-hydroxyquinoline; a complex including Alq₃; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present disclosure may be a front side emission type, a back side emission type, or a double side emission type according to the used material.

In addition, the compound according to the present disclosure may be included in an organic solar cell or an organic transistor in addition to an organic light emitting device.

The preparation of the compound represented by Chemical Formula 1 according to the present disclosure and the organic light emitting device containing the same will be described in detail in the following examples. However, these examples are presented for illustrative purposes only, and are not intended to limit the scope of the present disclosure.

### PREPARATION EXAMPLES

### Preparation Example 1: Preparation of Compound I

### Step 1) Preparation of Compound I'

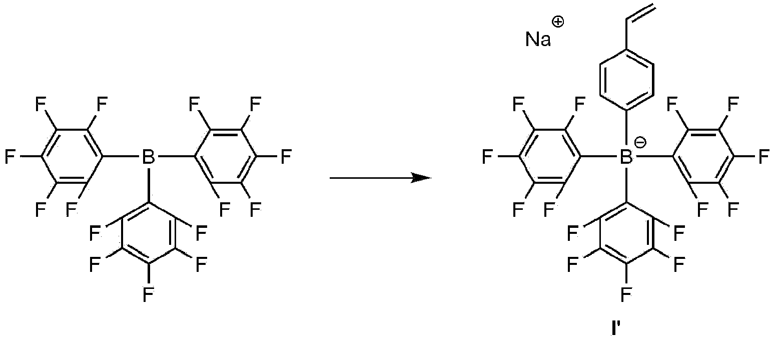

Mg (193 mg, 7.92 mmol), I₂(4 mg) and THF (10 mL) were placed in a 100 mL round bottom flask under a nitrogen atmosphere, and stirred for 30 minutes. 4-Bromostyrene (1.04 mL, 7.92 mmol) was added thereto, and the mixture was stirred for a day while a 30°C water bath was placed under the round bottom flask. Dissolution of Mg was identified by the solution becoming black. Ether (5 mL) was added thereto to dilute the reaction solution. Tris(pentafluorophenyl)borane (1 g, 3.96 mmol) was dissolved in ether (5 mL) and slowly added to the reaction solution for 30 minutes. The solution was stirred for a day. Na₂CO₃(0.1 M, 80 mL, 8.0 mmol) was slowly added to the reaction solution. The organic solvent was extracted using ethyl acetate (20 mL × 3), and residual water was removed with MgSO₄. In order to additionally remove residual water and impurities, the result was distilled with benzene using Dean-stark. When approximately 10 mL of the solvent was left, the solution was cooled and filtered to give Compound I' (1.6 g, yield: 64 %).

### Step 2) Preparation of Compound I

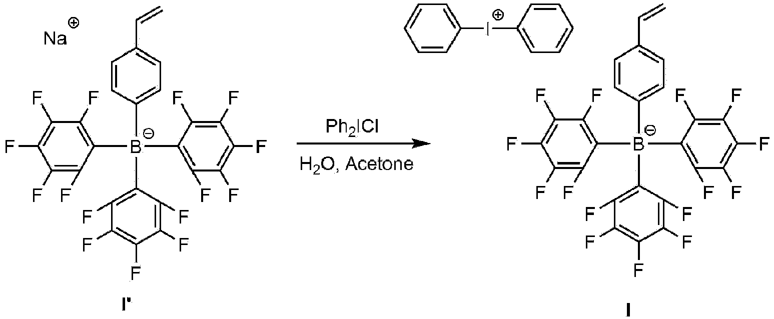

Compound I' (100 mg, 0.16 mmol), distilled water (10 mL) and Ph₂ICl(60 mg, 0.19 mmol) were placed in a 25 mL round bottom flask, and stirred for 1 hour. Acetone (15 mL) was added to the reaction solution to cause precipitation, and the precipitate was filtered and dried to give Compound I (140 mg, yield: 100%).
MS: [M-H]⁻ = 615 (negative mode)
MS: [M+H]⁺ = 281 (positive mode)

### Preparation Example 2: Preparation of Compound II

### Step 1) Preparation of Compound II'

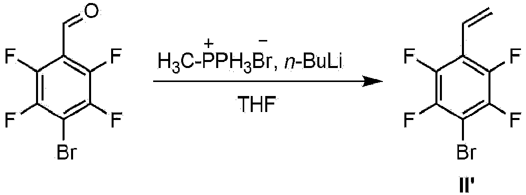

Methyltriphenyl potassium bromide (13.90 g, 38.91 mmol) and THF (100 mL) were placed in a 250 mL round bottom flask, and stirred at 0°C for 30 minutes. n-BuLi(15.6 mL, 38.91 mmol, 2.5 M in hexane) was slowly added to the reaction solution, and stirred at 0°C for 30 minutes. 4-Formyl-2,3,5,6-tetrafluoro-1-bromobenzene (5.0 g, 19.47 mmol, 30 mL in THF) was slowly added to the reaction solution at 0°C. The reaction solution was stirred while gradually raising the temperature to room temperature. After 3 hours, ether (100 mL) and saturated NH₄Cl solution (400 mL) were added to the reaction solution. The organic solvent was extracted with ether (200 mL×2) and the residual water was removed with MgSO₄. The resulting material was subjected to column chromatography with ethyl acetate:hexane = 1:9 (v:v) to give Compound II' (1.29 g, yield: 26%).

### Step 2) Preparation of Compound II"

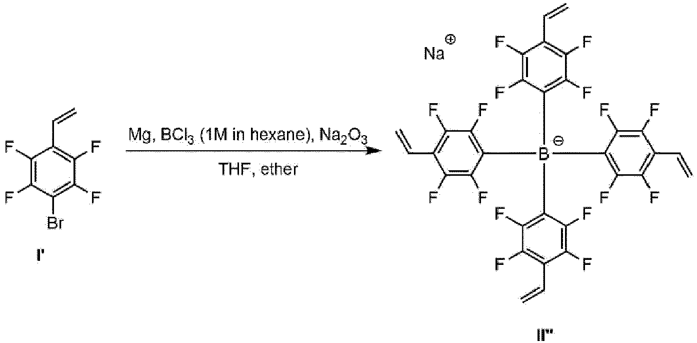

Mg (95 mg, 3.92 mmol), THF (10 mL) and I₂(4 mg) were placed in a 25 mL round bottom flask, and stirred. Compound I' (1.0 g, 3.92 mmol) was added to the reaction solution, and stirred at room temperature. After 10 hours, complete dissolution of Mg was identified by the solution becoming black, and ether (10 mL) and BCl₃(1.3 mL, 1.3 mmol, 1M in hexane solution) were added over 30 minutes. After stirring the reaction solution for a day, Na₂CO₃ (30 mL, 3.0 mmol, 0.1 M in H₂O) was added. The synthesized material was extracted with ethyl acetate (10 mL × 3), and then the residual water was removed with MgSO₄. After removing all the solvent, water was completely removed with Dean-stark using benzene, and the solids were filtered to give Compound II" (340 mg, yield: 28%).

### Step 3) Preparation of Compound II

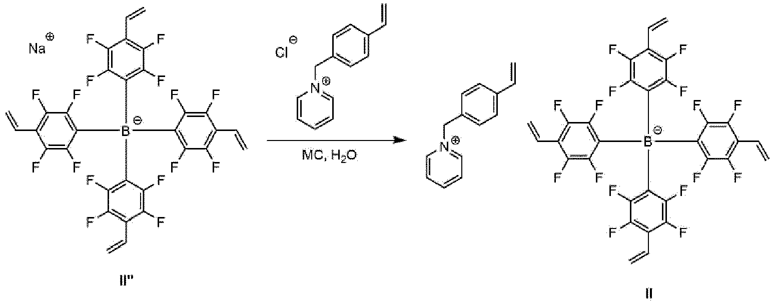

Compound II" (200 mg, 0.27 mmol), 1-(4-vinylbenzyl)pyridin-1-ium chloride (69 mg, 0.30 mmol), H₂O (10 mL) and methylene chloride (10 mL) were placed in a 25 mL round bottom flask, and vigorously stirred for 30 minutes. The organic solvent was extracted with ether (10 mL × 3) and the residual water was removed with MgSO₄. The solvent was removed and dried in vacuo to give Compound II (247 mg, yield: 100%).
MS: [M-H]⁻ = 711 (negative mode)
MS: [M+H]⁺ = 196 (positive mode)

### Preparation Example 3: Preparation of Compound III

### Step 1) Preparation of Compound III'

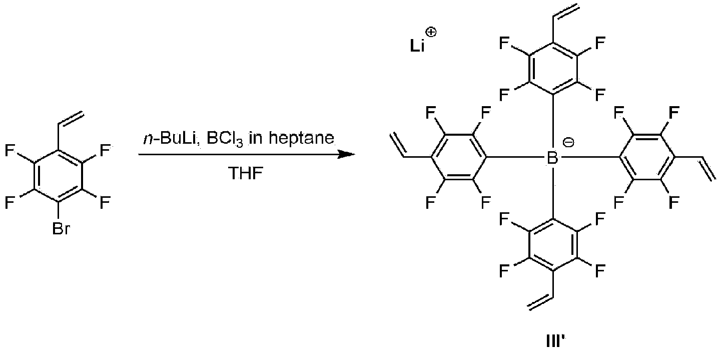

1-Bromo-2,3,5,6-tetrafluoro-4-vinylbenzene (2 g, 7.84 mmol) was added to THF (20 mL) in a 50 mL round bottom flask, and stirred at -78°C for 30 minutes. n-BuLi (3.45 mL, 8.63 mmol, 2.5 M in hexane) was slowly added to the solution, and stirred at -78°C for 30 minutes. BCl₃ (2.6 mL, 2.61 mmol, 1 M in hexane solution) was added to the reaction solution -78°C over 15 minutes. The reaction solution was stirred for a day while slowly raising the temperature to room temperature, and then water (30 mL) was added. The synthesized material was extracted with ethyl acetate (10 mL × 3), and then all solvent was removed. Water was completely removed with Dean-stark using benzene, and the solids were filtered to give Compound III" (800 mg, yield: 43%).

### Step 2) Preparation of Compound III

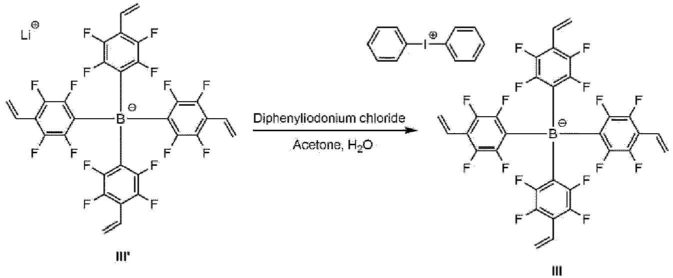

Compound III" (400 mg, 0.56 mmol), diphenyliodonium chloride (176 mg, 0.56 mmol), water (10 mL) and acetone (10 mL) were placed in a 25 mL round bottom flask, and vigorously stirred for 30 minutes. The result was extracted using dichloromethane (10 mL × 3), and then dried after removing the solvent to give Compound III (552 mg, yield: 100%)
MS: [M-H]⁻ = 711 (negative mode)
MS: [M+H]⁺ = 281 (positive mode)

### Preparation Example 4: Preparation of compound IV

### Step 1) Preparation of Compound IV'

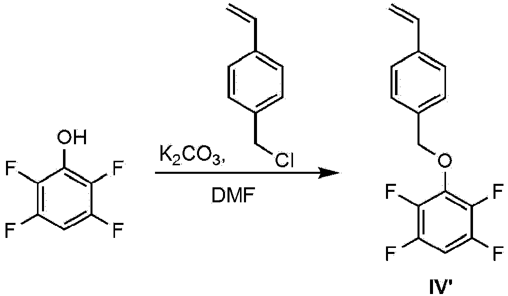

Potassium carbonate (10.4 g, 75.3 mmol) was placed in a 500 mL round bottom flask, to which DMF (200 ml) was added. To the flask, 2,3,5,6-tetrafluorophenol (10.0 g, 60.22 mmol) was added, and the mixture was stirred at 60°C for 30 minutes. 4-Vinylbenzyl chloride (7.66 g, 50.18 mmol) was slowly added to the reaction solution and stirred at 60°C for 16 hours. Then, water (300 mL) and ethyl acetate (200 ml) were added. The organic layer was extracted with ethyl acetate (200 mL × 2) and the residual water was removed with MgSO₄. The resulting material was subjected to column chromatography from ethyl acetate:hexane = 1:9 (v:v) to give Compound IV' (11.2 g, yield: 79%).

### Step 2) Preparation of compound IV"

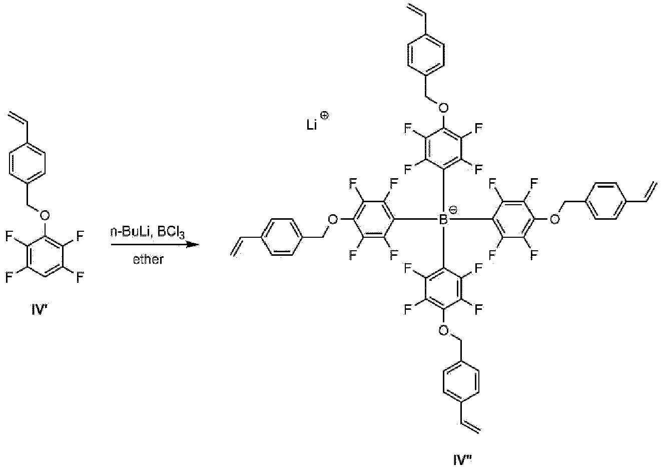

Compound IV' (10 g, 35.43 mmol) was placed in a 250 ml round bottom flask, to which ether (130 ml) was added, and the mixture was stirred. The reaction solution was cooled to -78°C, and stirred for 30 minutes. n-BuLi (17 ml, 42.52 mmol, 2.5 M in hexane) was slowly injected thereto over 30 minutes. Then, the result was stirred for 1 hour. BCl₃ (8.15 ml, 8.15 mmol, 1 M in hexane) was slowly added over 30 minutes. The temperature of the reaction solution was slowly raised to room temperature. After stirring the reaction solution for a day, water (200 mL) was added thereto. The synthesized material was extracted using ether (100 mL × 3), and all the solvent was removed. After that, water was completely removed with Dean-stark using benzene, and the solids were filtered to give Compound IV" (6.2 g, yield: 66%).

### Step 3) Preparation of Compound IV

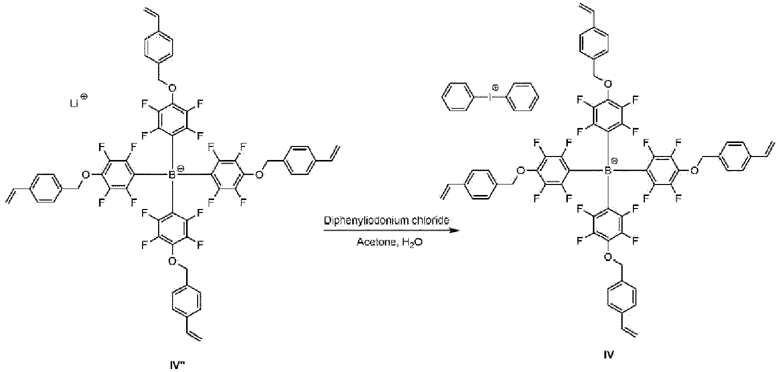

Compound IV" (6.2 g, 5.42 mmol), diphenyl iodonium chloride (2.57 g, 8.13 mmol), water (50 mL) and acetone (10 mL) were placed in a 25 mL round bottom flask, and vigorously stirred for 30 minutes. The organic solvent was extracted with methylene chloride (20 mL × 3) and the solvent was removed. The resulting material was subjected to column chromatography from methylene chloride: acetone = 9:1 (v:v) to give Compound IV (5.0 g, yield: 65%).
MS: [M-H]⁻ = 1135 (negative mode)
MS: [M+H]+ = 281 (positive mode)

### EXAMPLES

### Example 1: Preparation of Compound 1

### Step 1) Preparation of Compound 1-1

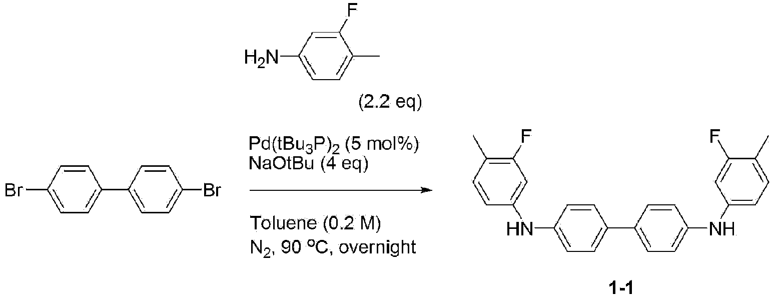

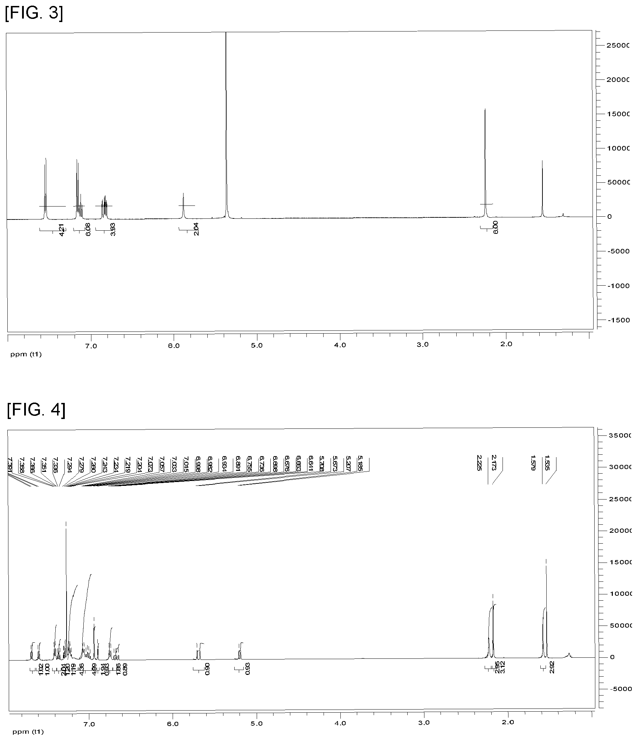

4,4'-Dibromobiphenyl (2 g, 6.4 mmol), Pd(tBu₃P)₂ (163.5 mg), NaOtBu (2.46 g, 25.6 mmol) were placed in a reactor which was purged with nitrogen. Toluene (32 mL) and 3-fluoro-4-methylaniline (1.6 mL, 14 mmol) were added thereto and stirred at 90°C overnight. The reaction mixture was worked up by adding ethyl acetate and brine, and then purified by column chromatography to give Compound 1-1, and NMR results (1H NMR (300 Hz, CD₂Cl₂)) are shown in FIG. 3.

### Step 2) Preparation of Compound 1

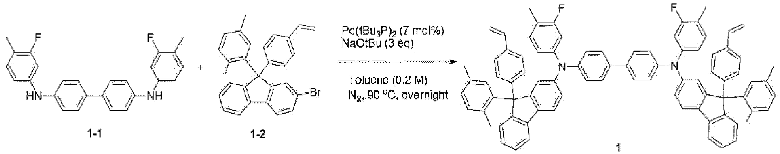

Compound 1-1 (0.6 g, 1.5 mmol), Pd(tBu₃P)₂ (54 mg) and NaOtBu (0.432 g, 4.5 mmol) were placed in a reactor which was purged with nitrogen. Toluene (7.5 mL) and Compound 1-2 (1.386 g, 3.07 mmol) were added thereto, and stirred at 90°C overnight. The reaction mixture was worked up by adding ethyl acetate and brine, and then purified by column chromatography to give Compound 1, and NMR results (1H NMR (300 Hz, CDCl₃)) are shown in FIG. 4.

### Example 2: Preparation of Compound 2

### Step 1) Preparation of Compound 2-1

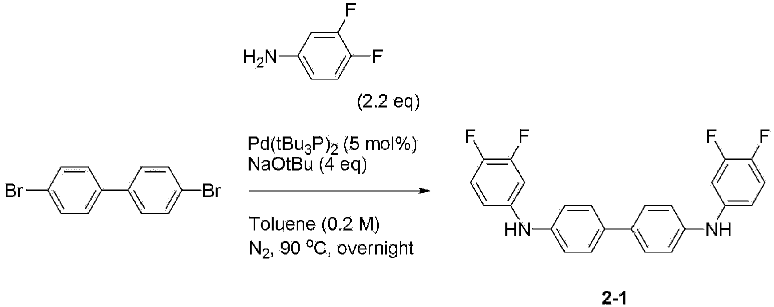

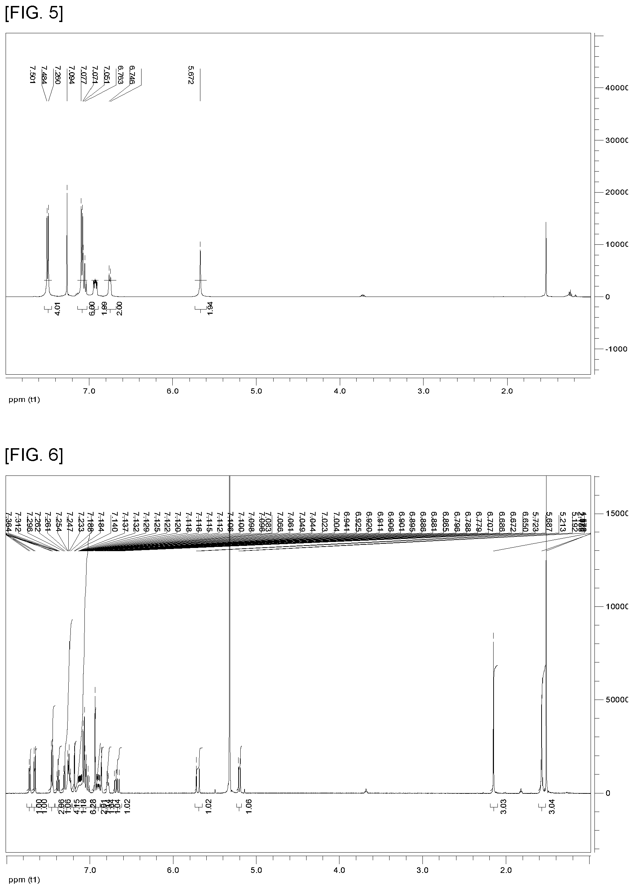

4,4'-dibromobiphenyl (2 g, 6.4 mmol), Pd(tBu₃P)₂ (163.5 mg) and NaOtBu (2.46 g, 25.6 mmol) were placed in a reactor which was purged with nitrogen. Toluene (32 mL) and 3,4-difluoroaniline (1.4 mL, 14.1 mmol) were added thereto, and stirred at 90°C overnight. The reaction mixture was worked up by adding ethyl acetate and brine, and then purified by column chromatography to give Compound 2-1, and NMR results (1H NMR (300 Hz, CDCl₃)) are shown in FIG. 5.

### Step 2) Preparation of Compound 2

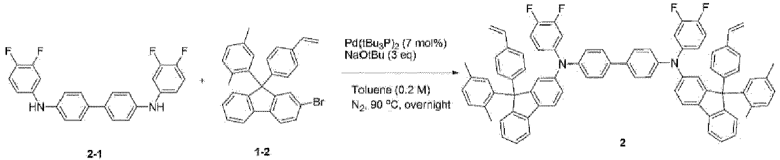

Compound 2-1 (1 g, 2.45 mmol), Pd(tBu₃P)₂ (87.6 mg, 0.17 mmol) and NaOtBu (0.706 g, 7.35 mmol) were placed in a reactor which was purged with nitrogen. Toluene (12 mL) and Compound 1-2 (2.27 mL, 5 mmol) were added thereto, and stirred at 90°C overnight. The reaction mixture was worked up by adding ethyl acetate and brine, and then purified by MPLC and then recrystallized with DCM to give Compound 2, and NMR results (1H NMR (300 Hz, CD₂Cl₂)) are shown in FIG. 6.

### Example 3: Preparation of Compound 3

### Step 1) Preparation of Compound 3-1

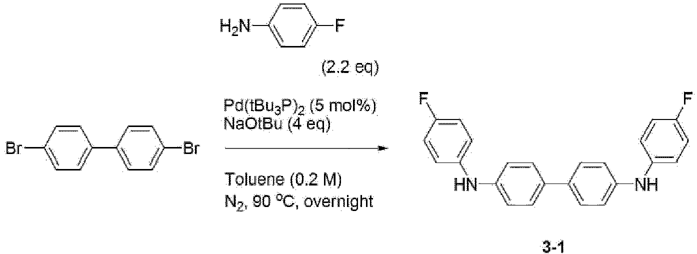

4,4'-dibromobiphenyl (0.5 g, 1.6 mmol), Pd(tBu₃P)₂ (40.9 mg) and NaOtBu (615 mg) were placed in a reactor which was purged with nitrogen. Toluene (8 mL) and 4-fluoroaniline (0.337 mL, 3.52 mmol) were added thereto, and stirred at 90°C overnight. The reaction mixture was worked up by adding ethyl acetate and brine, and then purified by column chromatography to give Compound 3-1.

### Step 2) Preparation of Compound 3

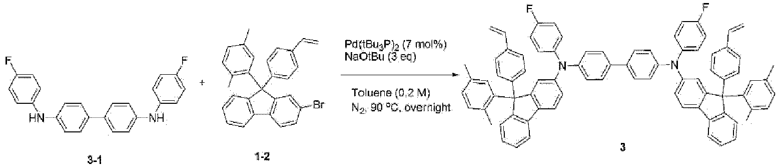

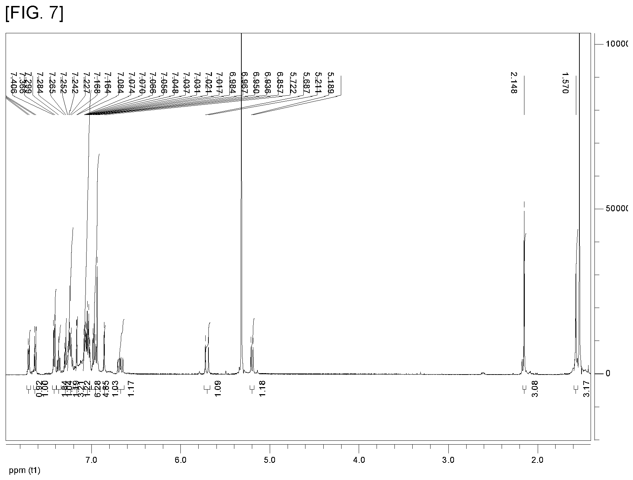

Compound 3-1 (0.32 g, 0.86 mmol), Pd(tBu₃P)₂(31 mg) and NaOtBu (0.248 g, 2.58 mmol) were placed in a reactor which was purged with nitrogen. Toluene (6 mL) and Compound 1-2 (900 mL, 1.76 mmol) were added thereto, and stirred at 90°C overnight. The reaction mixture was worked up by adding ethyl acetate and water, and then purified by column chromatography to give Compound 3, and NMR results (1H NMR (300 Hz, CDCl₃)) are shown in FIG. 7.

### Example 4: Preparation of Compound 4

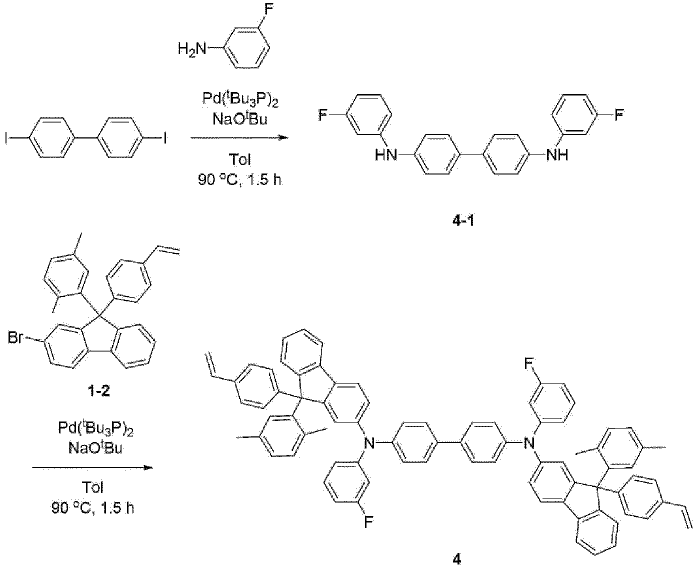

Diiodobiphenyl (5.00 g, 1.0 eq.), NaOtBu(7.10 g, 6.0 eq.), toluene (200 mL), and 3-fluoroaniline (2.39 mL, 2.02 eq.) were placed in a round bottom flask. After nitrogen gassing, the temperature was raised to 90°C. Pd(tBu₃P)₂ (0.250 g, 4 mol%) was added thereto, and then stirred at 90°C for 1.5 hours. The reaction mixture was worked up by adding ethyl acetate and brine, and then purified by column chromatography to give Compound 4-1.

Compound 4-1 (1.0 eq.) and Compound 1-2 (11.4 g, 2.05 eq.) were placed in a round bottom flask, and NaOtBu (7.10 g, 6.0 eq.), toluene (200 mL) and Pd(tBu₃P)₂ (0.25 g, 4 mol%) were added and then stirred at 90°C for 1.5 hours. Then, the resulting material was purified by column chromatography to give Compound 4.
MS: [M+H]⁺ = 1113

### Example 5: Preparation of Compound 5

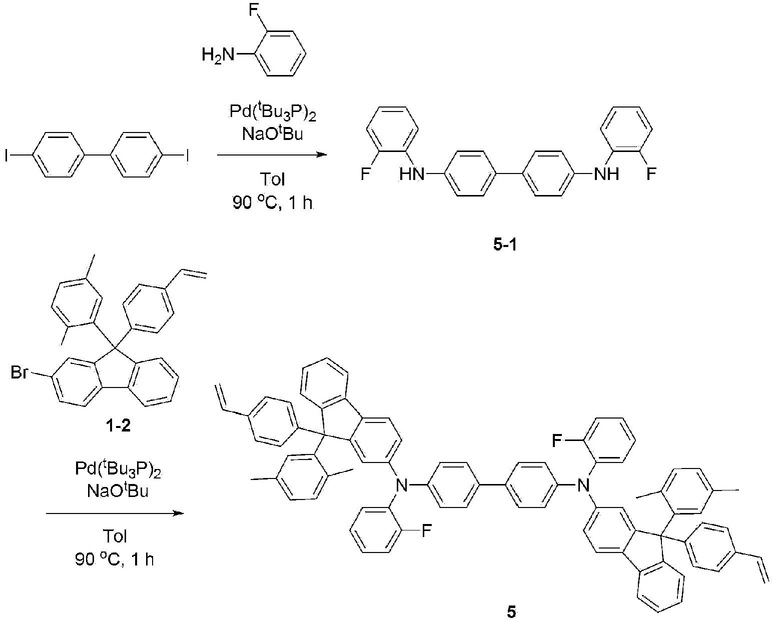

Diiodobiphenyl (5.00 g, 1.0 eq.), NaOtBu(7.10 g, 6.0 eq.), toluene (200 mL), and 2-fluoroaniline (2.40 mL, 2.02 eq.) were placed in a round bottom flask. After nitrogen gassing, the temperature was raised to 90°C. Pd(tBu₃P)₂ (0.250 g, 4 mol%) was added thereto, and then stirred at 90°C for 1 hour. The reaction mixture was worked up by adding ethyl acetate and brine, and then purified by column chromatography to give Compound 5-1.

Compound 5-1 (1.0 eq.) and Compound 1-2 (11.4 g, 2.05 eq.) were placed in a round bottom flask, and NaOtBu (7.10 g, 6.0 eq.), toluene (200 mL) and Pd(tBu₃P)₂ (0.25 g, 4 mol%) were added thereto, and then stirred at 90°C for 1 hours. Then, the resulting material was purified by column chromatography to give Compound 5.
MS: [M+H]⁺ = 1113

### Example 6: Preparation of Compound 6

### Step 1) Preparation of Compound 6-1

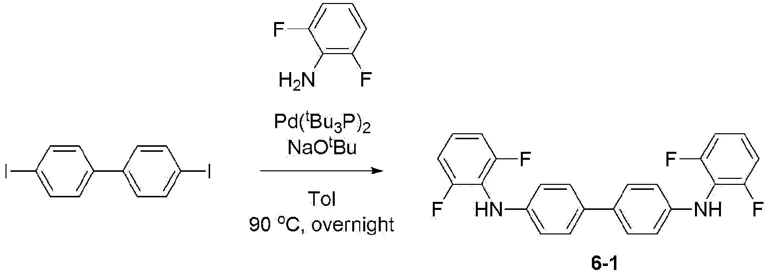

Diiodobiphenyl (6.00 g, 1.0 eq.), 2,6-difluoroaniline (4.20 g, 2.2 eq.), NaOtBu (4.26 g, 3.0 eq) and toluene (85 mL) were placed in a round bottom flask. After nitrogen gassing, the temperature was raised to 90°C. Pd(tBu₃P)₂ was added thereto, and then stirred at 90°C overnight.

### Step 2) Preparation of Compound 6

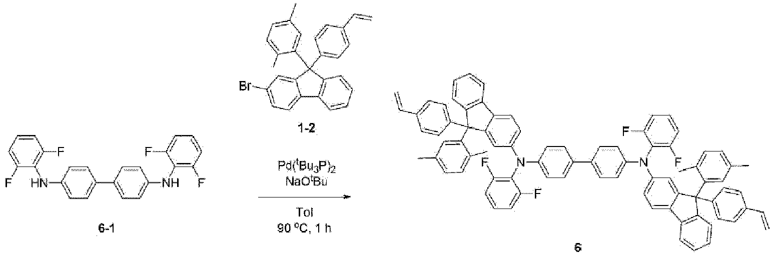

Compound 6-1 (1.30 g, 1.0 eq.), Compound 1-2 (3.02 g, 2.1 eq.), NaOtBu (0.918 g, 3.0 eq.) and toluene (30 mL) were placed in a round bottom flask. After nitrogen gassing, the temperature was raised to 90°C. Pd(tBu₃P)₂ (0.114 g, 7 mol%) was added thereto, and then stirred at 90°C for 1 hour. The reaction mixture was then purified by column chromatography to give Compound 6.
MS: [M+H]⁺ = 1149

### Example 7: Preparation of Compound 7

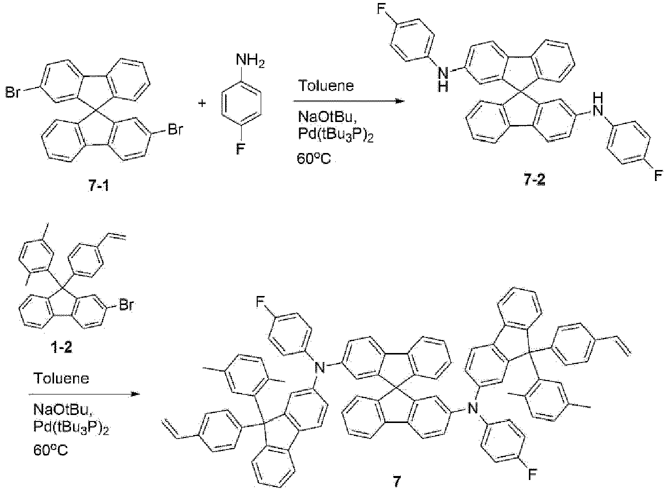

Compound 7-1 (1.0 eq.), NaOtBu (7.10 g, 6.0 eq.), toluene (200 mL) and 4-fluoroaniline (2.40 mL, 2.02 eq.) were placed in a round bottom flask. After nitrogen gassing, the temperature was raised to 60°C. Pd(tBu₃P)₂ (0.250 g, 4 mol%) was added thereto, and then stirred at 60°C for 1 hour. The reaction mixture was worked up by adding ethyl acetate and brine, and then purified by column chromatography to give Compound 7-2.

Compound 7-2 (1.0 eq.) and Compound 1-2 (11.4 g, 2.05 eq.) were placed in a round bottom flask, and NaOtBu (7.10 g, 6.0 eq.), toluene (200 mL) and Pd(tBu₃P)₂ (0.25 g, 4 mol%) were added thereto, and then stirred at 60°C for 1 hour. Then, the resulting material was purified by column chromatography to give Compound 7.
MS: [M+H]⁺ = 1276

### Example 8: Preparation of Compound 8

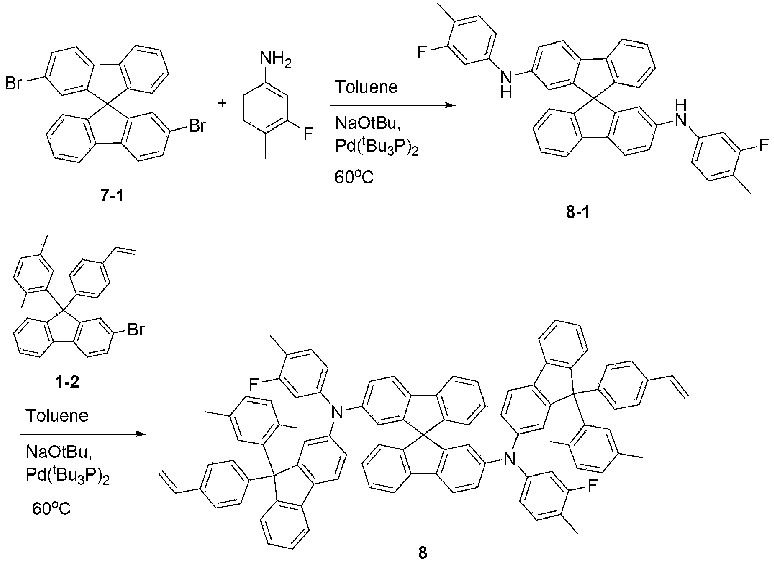

Compound 7-1 (1.0 eq.), NaOtBu (7.10 g, 6.0 eq.), toluene (200 mL) and 3-fluoro-4-methylaniline (2.02 eq.) were placed in a round bottom flask. After nitrogen gassing, the temperature was raised to 60°C. Pd(tBu₃P)₂ (0.250 g, 4 mol%) was added thereto, and then stirred at 60°C for 1 hour. The reaction mixture was worked up by adding ethyl acetate and brine, and then purified by column chromatography to give Compound 8-1.

Compound 8-1 (1.0 eq.) and Compound 1-2 (11.4 g, 2.05 eq.) were placed in a round bottom flask, and NaOtBu (7.10 g, 6.0 eq.), toluene (200 mL) and Pd(tBu₃P)₂ (0.25 g, 4 mol%) were added thereto, and then stirred at 60°C for 1 hour. Then, the resulting material was purified by column chromatography to give Compound 8.
MS: [M+H]⁺ = 1304

### Example 9: Preparation of Compound 9

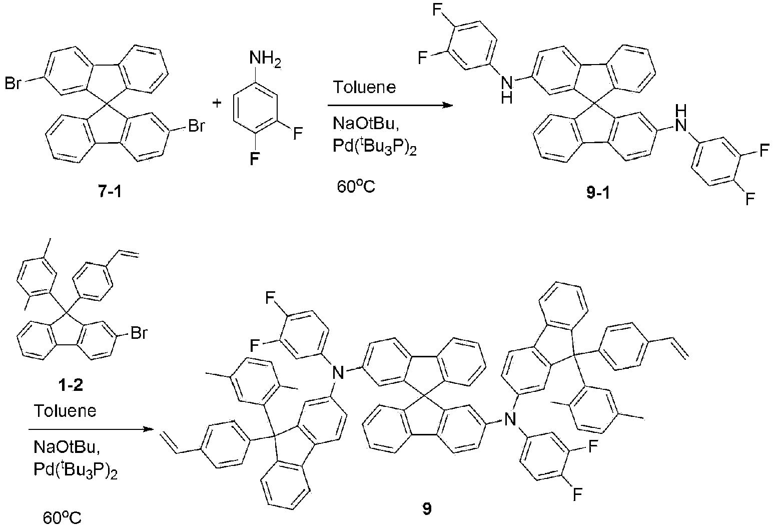

Compound 7-1 (1.0 eq.), NaOtBu (7.10 g, 6.0 eq.), toluene (200 mL) and 3,4-difluoroaniline (2.02 eq.) were placed in a round bottom flask. After nitrogen gassing, the temperature was raised to 60°C. Pd(tBu₃P)₂ (0.250 g, 4 mol%) was added thereto, and then stirred at 60°C for 1 hour. The reaction mixture was worked up by adding ethyl acetate and brine, and then purified by column chromatography to give Compound 9-1.

Compound 9-1 (1.0 eq.) and Compound 1-2 (11.4 g, 2.05 eq.) were placed in a round bottom flask, and NaOtBu (7.10 g, 6.0 eq.), toluene (200 mL) and Pd(tBu₃P)₂ (0.25 g, 4 mol%) were added thereto, and then stirred at 60°C for 1 hour. Then, the resulting material was purified by column chromatography to give Compound 9.
MS: [M+H]⁺ = 1312

### Example 10: Preparation of Compound 10

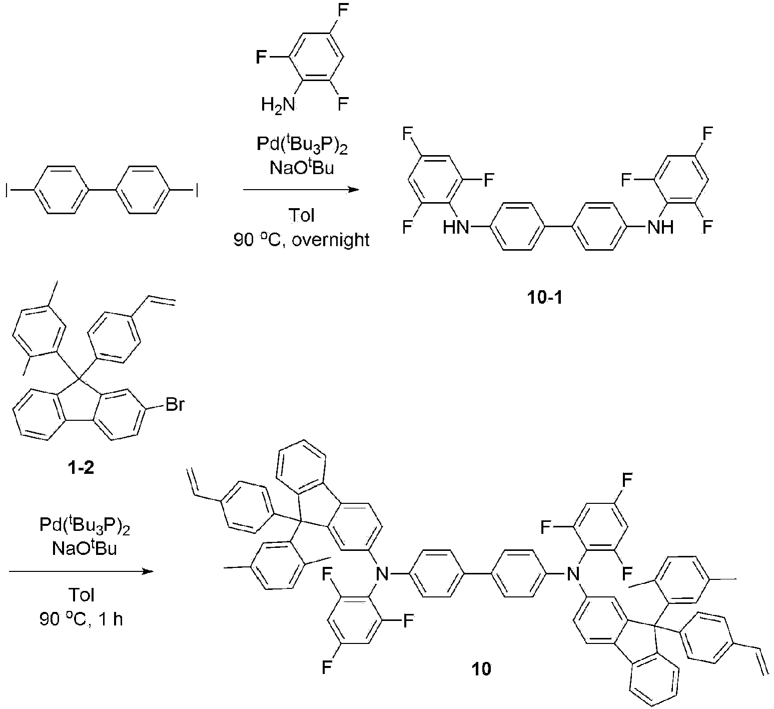

Diiodobiphenyl (4.00 g, 1.0 eq.), 2,4,6-trifluoroaniline (2.2 eq.), NaOtBu (3.79 g, 4.0 eq) and toluene (100 mL) were placed in a round bottom flask. After nitrogen gassing, the temperature was raised to 90°C. Pd(tBu₃P)₂ (0.25 g, 5 mol%) was added thereto, and then stirred at 90°C overnight. The reaction mixture was worked up by adding ethyl acetate and brine, and then purified by column chromatography to give Compound 10-1.

Compound 10-1 (1.0 eq.) and Compound 1-2 (2.05 eq.) were placed in a round bottom flask, and NaOtBu (6.0 eq.), toluene (100 mL) and Pd(tBu₃P)₂ (0.25 g, 5 mol%) were added thereto, and then stirred at 90°C for 1 hour. Then, the resulting material was purified by column chromatography to give Compound 10.
MS: [M+H]⁺ = 1185

### Example 11: Preparation of Compound 11

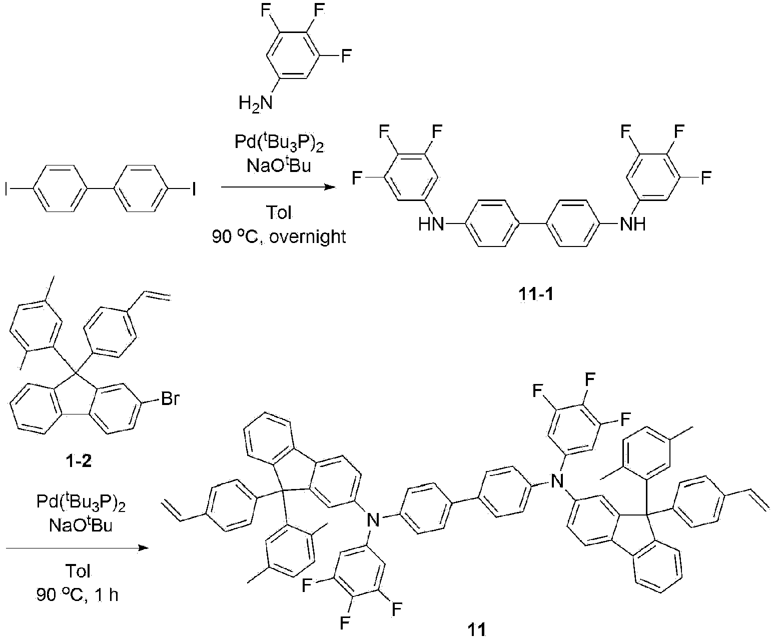

Diiodobiphenyl (4.00 g, 1.0 eq.), 3,4,5-trifluoroaniline (2.2 eq.), NaOtBu (3.79 g, 4.0 eq) and toluene (100 mL) were placed in a round bottom flask. After nitrogen gassing, the temperature was raised to 90°C. Pd(tBu₃P)₂ (0.25 g, 5 mol%) was added thereto, and then stirred at 90°C overnight. The reaction mixture was worked up by adding ethyl acetate and brine, and then purified by column chromatography to give Compound 11-1.

Compound 11-1 (1.0 eq.) and Compound 1-2 (2.05 eq.) were placed in a round bottom flask, and NaOtBu (6.0 eq.), toluene (100 mL) and Pd(tBu₃P)₂ (0.25 g, 5 mol%) were added thereto, and then stirred at 90°C for 1 hour. Then, the resulting material was purified by column chromatography to give Compound 11.
MS: [M+H]⁺ = 1185

### Example 12: Preparation of Compound 12

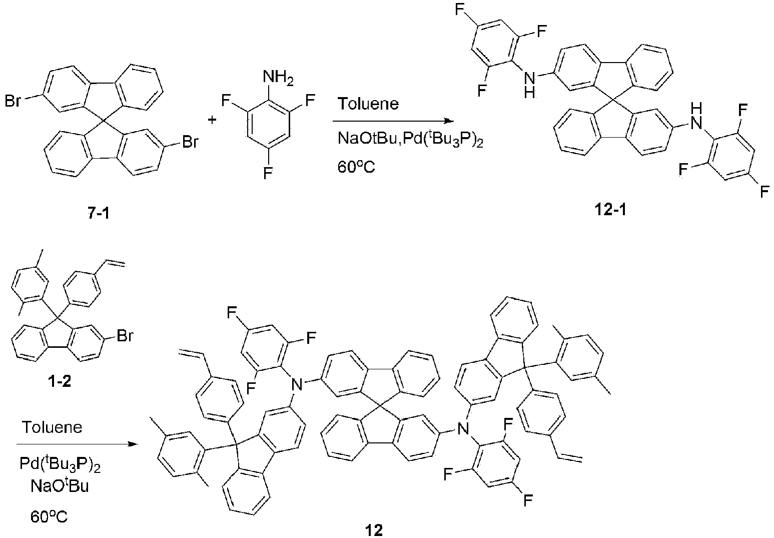

Compound 7-1 (1.0 eq.), NaOtBu (7.10 g, 6.0 eq.), toluene (200 mL) and 2,4,6-trifluoroaniline (2.02 eq.) were placed in a round bottom flask. After nitrogen gassing, the temperature was raised to 60°C. Pd(tBu₃P)₂ (5 mol%) was added thereto, and then stirred at 60°C for 1 hour. The reaction mixture was worked up by adding ethyl acetate and brine, and then purified by column chromatography to give Compound 12-1.

Compound 12-1 (1.0 eq.) and Compound 1-2 (2.05 eq.) were placed in a round bottom flask, and NaOtBu (6.0 eq.), toluene (100 mL) and Pd(tBu₃P)₂ (5 mol%) were added thereto, and then stirred at 60°C for 1 hour. Then, the resulting material was purified by column chromatography to give Compound 12.
MS: [M+H]⁺ = 1348

### Example 13: Preparation of Compound 13

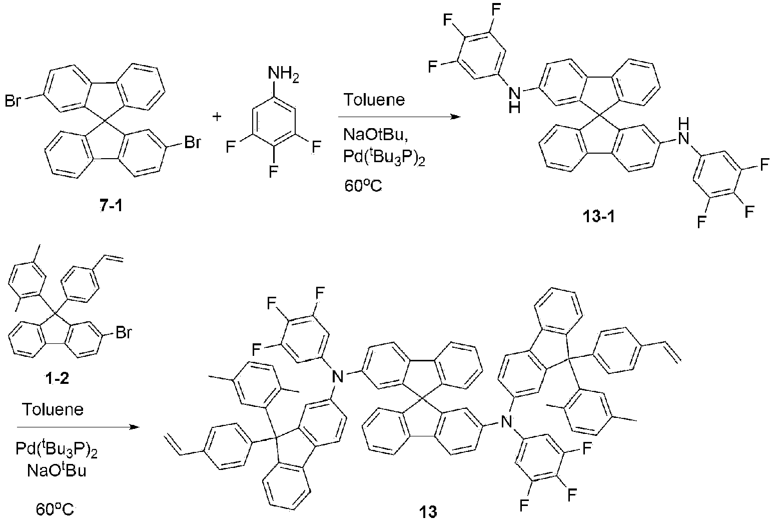

Compound 7-1 (1.0 eq.), NaOtBu (7.10 g, 6.0 eq.), toluene (200 mL) and 3,4,5-trifluoroaniline (2.02 eq.) were placed in a round bottom flask. After nitrogen gassing, the temperature was raised to 60°C. Pd(tBu₃P)₂ (4 mol%) was added thereto, and then stirred at 60°C for 1 hour. The reaction mixture was worked up by adding ethyl acetate and brine, and then purified by column chromatography to give Compound 13-1.

Compound 13-1 (1.0 eq.) and Compound 1-2 (2.05 eq.) were placed in a round bottom flask, and NaOtBu (6.0 eq.), toluene (100 mL) and Pd(tBu₃P)₂ (5 mol%) were added thereto, and then stirred at 60°C for 1 hour. Then, the resulting material was purified by column chromatography to give Compound 13.
MS: [M+H]⁺ = 1348

### Example 14: Preparation of Compound 14

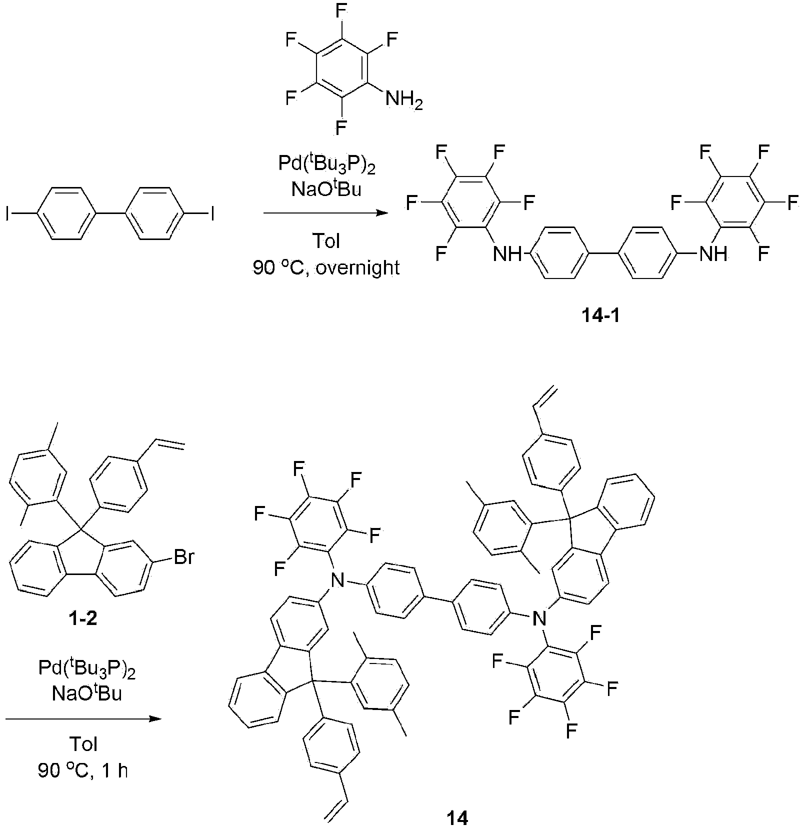

Diiodobiphenyl (6.00 g, 1.0 eq.), 2,3,4,5,6-pentafluoroaniline (2.2 eq.), NaOtBu (4.26 g, 3.0 eq) and toluene (120 mL) were placed in a round bottom flask. After nitrogen gassing, the temperature was raised to 90°C. Pd(tBu₃P)₂ (4 mol%) was added thereto, and then stirred at 90°C for 1 hour. The reaction mixture was worked up by adding ethyl acetate and brine, and then purified by column chromatography to give Compound 14-1.

Compound 14-1 (1.0 eq.) and Compound 1-2 (2.05 eq.) were placed in a round bottom flask, and NaOtBu (6.0 eq.), toluene (100 mL) and Pd(tBu₃P)₂ (5 mol%) were added thereto, and then stirred at 90°C for 1 hour. Then, the resulting material was purified by column chromatography to give Compound 14.
MS: [M+H]⁺ = 1257

### Example 15: Preparation of Compound 15

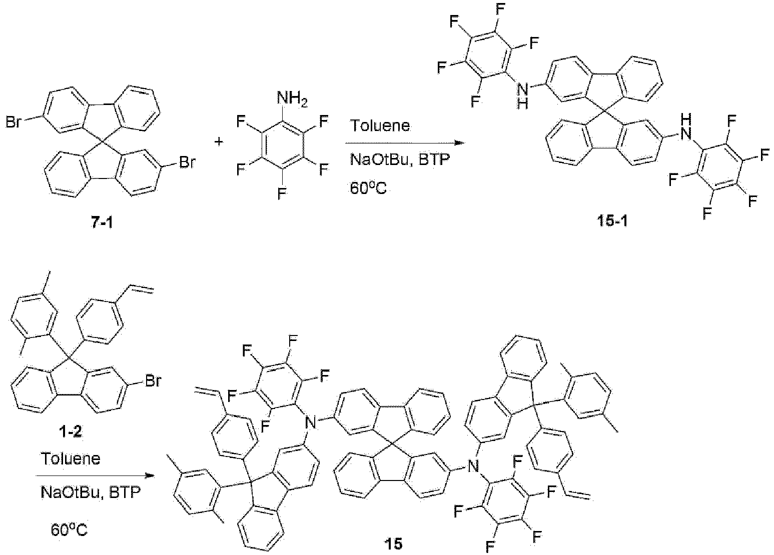

Compound 7-1 (1.0 eq.), NaOtBu (7.10 g, 6.0 eq.), toluene (200 mL) and 2,3,4,5,6-pentafluoroaniline (2.02 eq.) were placed in a round bottom flask. After nitrogen gassing, the temperature was raised to 60°C. Pd(tBu₃P)₂ (4 mol%) was added thereto, and then stirred at 60°C for 1 hour. The reaction mixture was worked up by adding ethyl acetate and brine, and then purified by column chromatography to give Compound 15-1.

Compound 15-1 (1.0 eq.) and Compound 1-2 (2.05 eq.) were placed in a round bottom flask, and NaOtBu (6.0 eq.), toluene (100 mL) and Pd(tBu₃P)₂ (5 mol%) were added thereto, and then stirred at 60°C for 1 hour. Then, the resulting material was purified by column chromatography to give Compound 15.
MS: [M+H]⁺ = 1419

### EXPERIMENTAL EXAMPLES

### Experimental Example 1

A glass substrate on which ITO (indium tin oxide) was coated as a thin film to a thickness of 1500 Å was put into distilled water in which a detergent was dissolved, and ultrasonically cleaned. At this time, a product manufactured by Fischer Co. was used as the detergent, and as the distilled water, distilled water filtered twice using a filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol and acetone, dried, and then the substrate was cleaned for 5 minutes and then transferred to a glove box.

On the transparent ITO electrode prepared as above, a 2 wt% cyclohexanone solution containing the compound 1 prepared in the previous Example 1 as a host and the compound III prepared in the previous Preparation Example 3 as a dopant, with the weight ratio of the host and the dopant being 8:2, was spin coated and heat treated at 230°C for 30 minutes to form a hole injection layer having a thickness of 400 Å. A 2 wt% toluene solution of Compound a-NPD below was spin-coated on the hole injection layer and heat-treated at 120°C for 10 minutes to form a hole transport layer having a thickness of 200 Å.

Subsequently, the result was transferred to a vacuum depositor, and then Compound A below and Compound B below were vacuum-deposited in a weight ratio of 9:1 on the hole transport layer to form a light emitting layer having a thickness of 300 Å. Compound C was vacuum deposited on the light emitting layer to form an electron injection and transport layer having a thickness of 400 Å. LiF and aluminum were sequentially deposited to have a thickness of 5 Å and 1,000 Å, respectively, on the electron injection and transport layer, thereby forming a cathode.

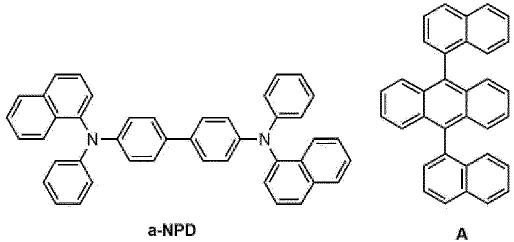

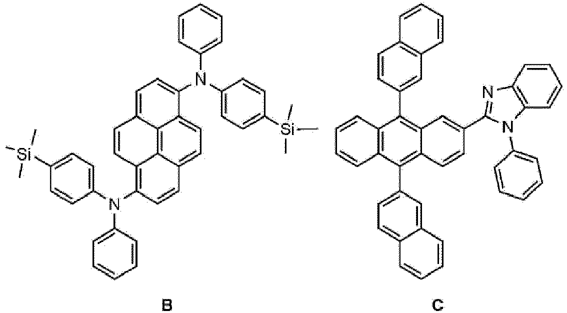

In the above-mentioned processes, the deposition rates of the organic materials were maintained at 0.4 to 1.0 Å/sec, the deposition rates of the LiF and the aluminum of the cathode were maintained at 0.3 Å /sec and 2 Å /sec, respectively, and the degree of vacuum during the deposition was maintained at 2×10⁻⁸ to 5×10⁻⁶ torr.

### Experimental Examples 2 to 15

The organic light emitting devices were manufactured in the same manner as in Experimental Example 1, except that the compounds shown in Table 1 below were used during the formation of the hole injection layer.

### Comparative Experimental Examples 1 to 3

The organic light emitting devices were manufactured in the same manner as in Experimental Example 1, except that the compounds shown in Table 1 below were used during the formation of the hole injection layer. Compounds CE1, CE2, and CE3 in Table 1 are as follows.

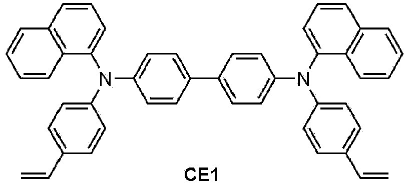

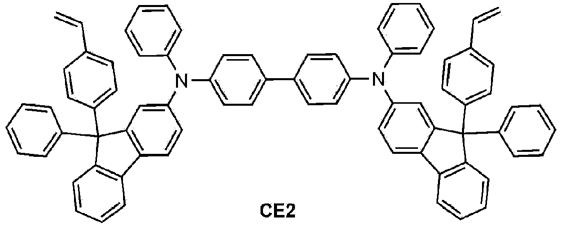

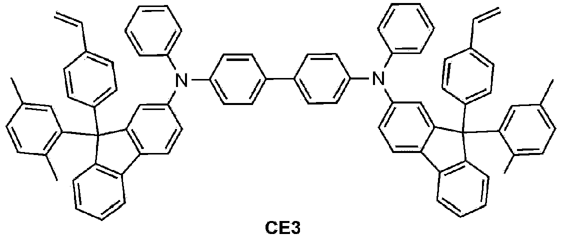

For the organic light emitting devices manufactured in the Experimental Examples and Comparative Experimental Examples, the driving voltage, luminous efficiency, power efficiency, external quantum efficiency (QE), luminance, and color coordinates were measured at a current density of 10 mA/cm², and the results are shown in Table 1 below. The external quantum efficiency was determined by (number of photons emitted)/(number of charge carriers injected), and the color coordinates are x and y coordinates based no C.I.E chromaticity diagram (Commission Internationale de L'Eclairage, 1931).

**Table 1**

| | Hole injection layer (host/dopant) | Driving voltage (V) | Emissi on efficien cy (cd/A) | Power efficien cy (lm/W) | QE (%) | Lumina nce (cd/m²) | ClEx | ClEy |
|---|---|---|---|---|---|---|---|---|
| Experimental Ex. 1 | Com. 1/ Com. III | 4.34 | 4.66 | 3.37 | 5.46 | 466 | 0.138 | 0.105 |
| Experimental Ex. 2 | Com. 2/ Com. IV | 4.38 | 4.68 | 3.35 | 5.45 | 468 | 0.137 | 0.105 |
| Experimental Ex. 3 | Com. 3/ Com. II | 4.38 | 4.78 | 3.43 | 5.69 | 478 | 0.137 | 0.106 |
| Experimental Ex. 4 | Com. 4/ Com. I | 4.31 | 4.62 | 3.37 | 5.43 | 462 | 0.137 | 0.110 |
| Experimental Ex. 5 | Com. 5/ Com. I | 4.29 | 4.68 | 3.42 | 5.66 | 468 | 0.138 | 0.105 |
| Experimental Ex. 6 | Com. 6/ Com. II | 4.33 | 4.55 | 3.30 | 5.38 | 455 | 0.139 | 0.100 |
| Experimental Ex. 7 | Com. 7/ Com. IV | 4.35 | 4.72 | 3.41 | 5.51 | 472 | 0.137 | 0.109 |
| Experimental Ex. 8 | Com. 8/ Com. II | 4.37 | 4.68 | 3.36 | 5.45 | 468 | 0.138 | 0.108 |
| Experimental Ex. 9 | Com. 9/ Com. II | 4.38 | 4.55 | 3.26 | 5.27 | 455 | 0.138 | 0.107 |
| Experimental Ex. 10 | Com. 10/ Com. I | 4.36 | 4.71 | 3.39 | 5.49 | 471 | 0.139 | 0.100 |
| Experimental Ex. 11 | Com. 11/ Com. IV | 4.40 | 4.59 | 3.28 | 5.35 | 459 | 0.138 | 0.105 |
| Experimental Ex. 12 | Com. 12/ Com. III | 4.39 | 4.57 | 3.27 | 5.33 | 457 | 0.137 | 0.105 |
| Experimental Ex. 13 | Com. 13/ Com. I | 4.42 | 4.62 | 3.28 | 5.36 | 462 | 0.137 | 0.106 |
| Experimental Ex. 14 | Com. 14/ Com. IV | 4.43 | 4.68 | 3.31 | 5.39 | 468 | 0.137 | 0.110 |
| Experimental Ex. 15 | Com. 15/ Com. II | 4.33 | 4.59 | 3.33 | 5.42 | 459 | 0.138 | 0.105 |
| Comparative Experimental Ex. 1 | Com. CE1/ Com. I | 4.90 | 4.15 | 2.66 | 4.60 | 415 | 0.137 | 0.105 |
| Comparative Experimental Ex. 2 | Com. CE2/ Com. II | 4.81 | 4.20 | 2.74 | 4.77 | 420 | 0.137 | 0.106 |
| Comparative Experimental Ex. 3 | Com. CE3/ Com. II | 4.62 | 4.28 | 2.91 | 4.82 | 428 | 0.137 | 0.106 |

### Description of Symbols

| | | | |
|---|---|---|---|
| 1: | substrate | 2: | anode |
| 3: | light emitting layer | 4: | cathode |
| 5: | hole injection layer | 6: | hole transport layer |
| 7: | light emitting layer | 8: | electron injection and transport layer |

## Claims

1. A compound represented by the following Chemical Formula 1: 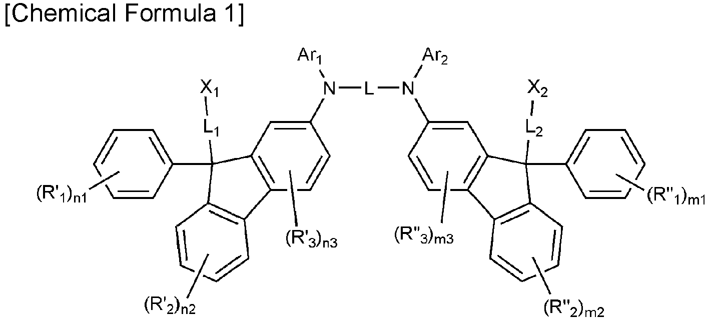 wherein
L is substituted or unsubstituted C₆₋₆₀ arylene, or substituted or unsubstituted C₂₋₆₀ heteroarylene containing any one or more heteroatoms selected from N, O and S;
L₁ and L₂ are each independently a single bond or methylene;
X₁ and X₂ are each independently a photocurable group or a thermosetting group independently represented by -L"-R", wherein L" is a single bond, -O-, -S-, - CH₂-, -CH₂O-, -OCH₂-, or -CH₂OCH₂-, and R" is any one of the following: 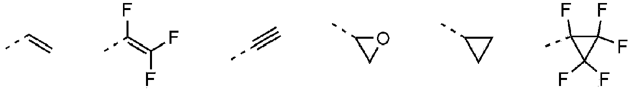 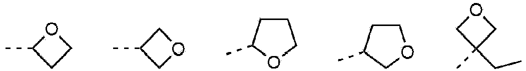 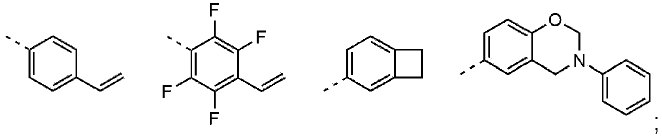
R'₁ to R'₃ and R"₁ to R"₃ are each independently hydrogen, deuterium, substituted or unsubstituted C₁₋₆₀ alkyl, substituted or unsubstituted C₁₋₆₀ alkoxy, substituted or unsubstituted C₆₋₆₀ aryl, or substituted or unsubstituted C₂₋₆₀ heteroaryl containing any one or more heteroatoms selected from N, O and S;
n1 to n3 and m1 to m3 are each independently an integer of 0 to 3; and
Ar₁ and Ar₂ are each independently a substituent group represented by the following Chemical Formula 2: 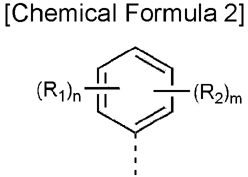 wherein
each R₁ is independently a halogen;
each R₂ is independently hydrogen, deuterium, or C₁₋₁₀alkyl; and
n is an integer of 1 to 5, and m is 0 or 1, provided that n+m is 5 or less.

2. The compound of claim 1, wherein L is phenylene, biphenyldiyl, or spirobifluorenediyl.

3. The compound of claim 1, wherein L is any one of the following:

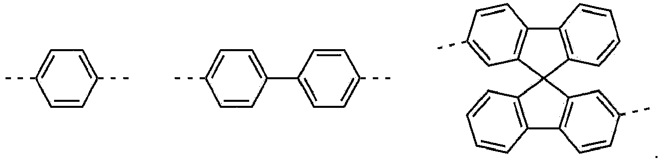

4. The compound of claim 1, wherein R₁ is fluoro.

5. The compound of claim 1, wherein R₂ is hydrogen, deuterium, or methyl.

6. The compound of claim 1, wherein Ar₁ and Ar₂ are each independently a substituent group represented by: 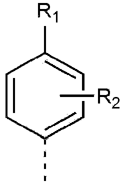 wherein
R₁ is C₁₋₁₀ alkyl, or a halogen; and
R₂ is hydrogen, deuterium, C₁₋₁₀ alkyl, or a halogen;
with the proviso that at least one of R₁ and R₂ is a halogen.

7. The compound of claim 1, wherein Ar₁ and Ar₂ are each independently any one of the following:

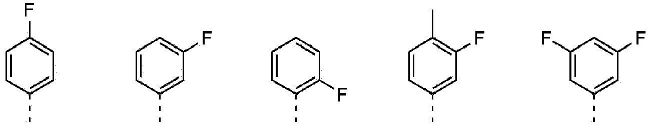

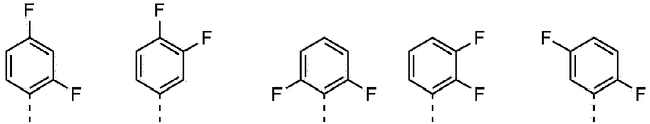

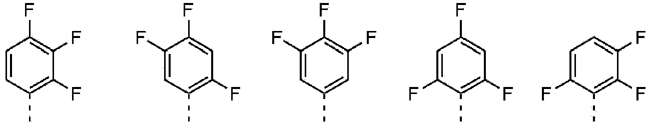

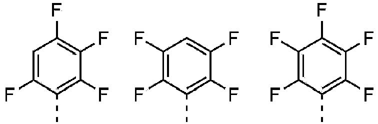

8. The compound of claim 1, wherein
R'₁ and R"₁ are each independently hydrogen or methyl; and
n1 and m1 are each independently an integer of 0 to 2.

9. The compound of claim 1, wherein R'₂, R'₃, R"₂ and R"₃ are hydrogen.

10. The compound of claim 1, which is any one of the following:

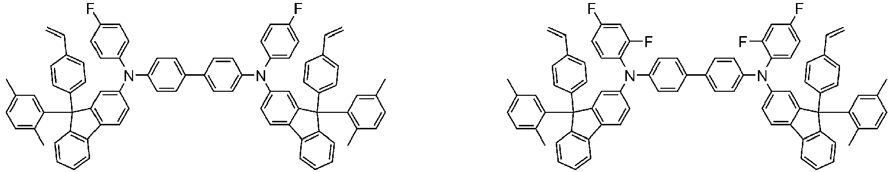

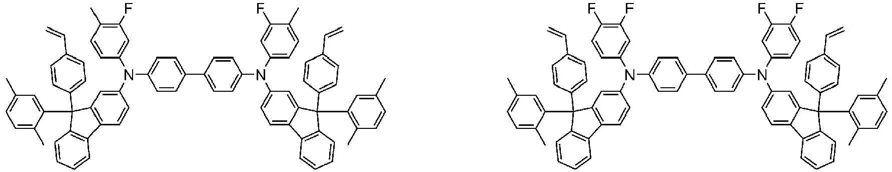

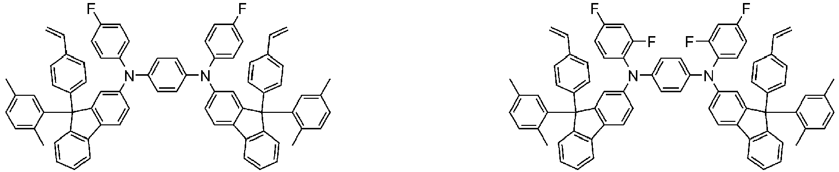

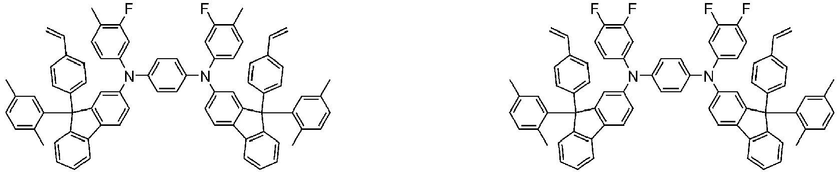

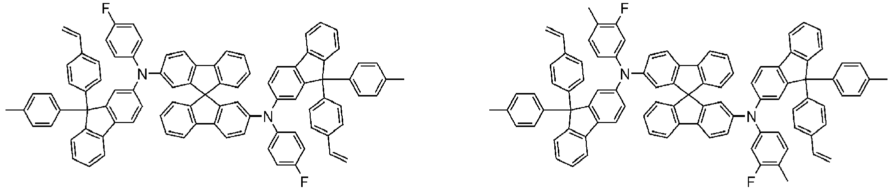

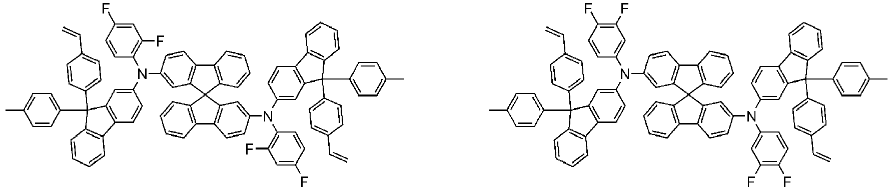

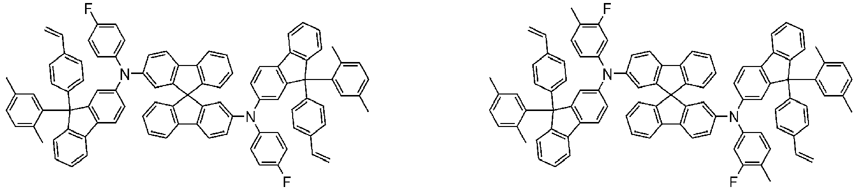

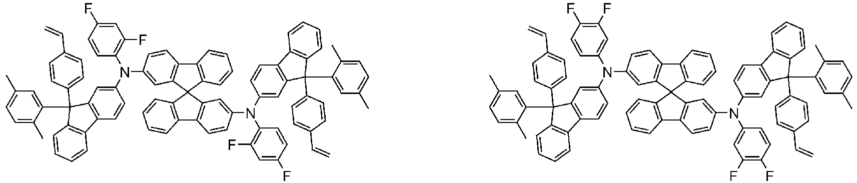

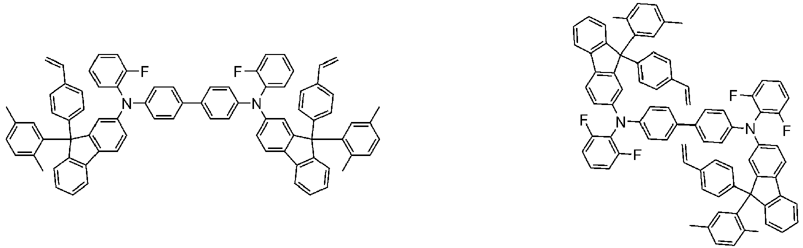

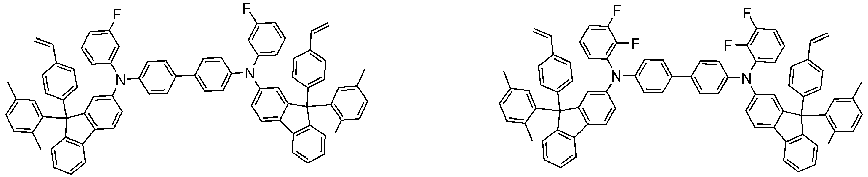

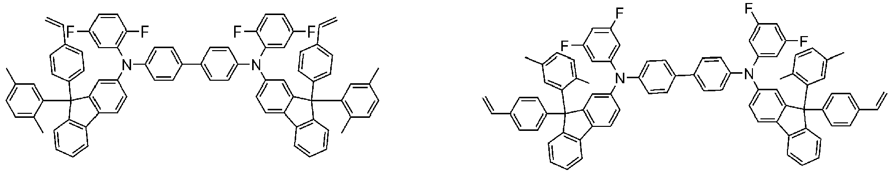

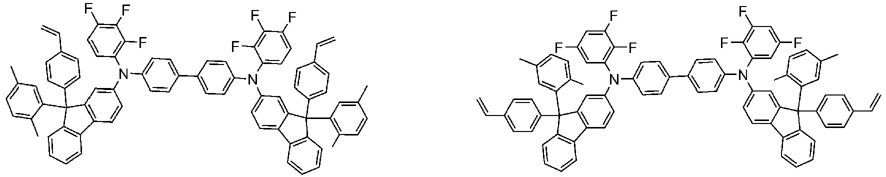

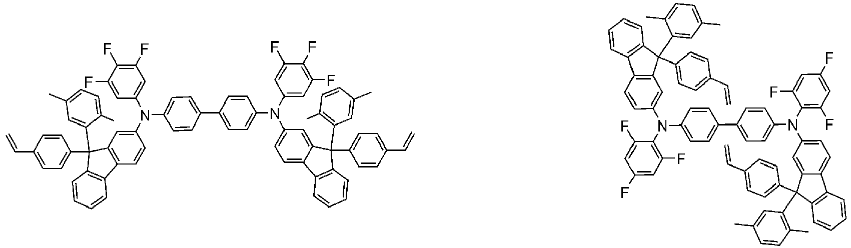

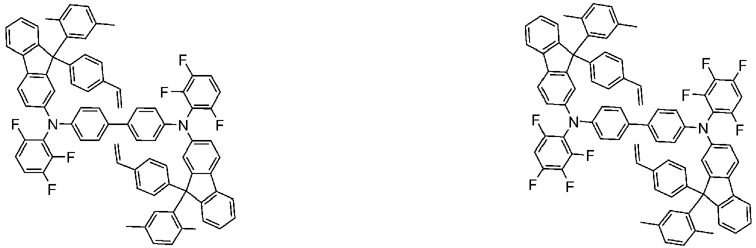

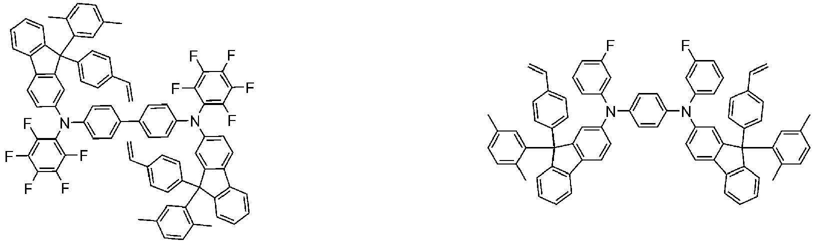

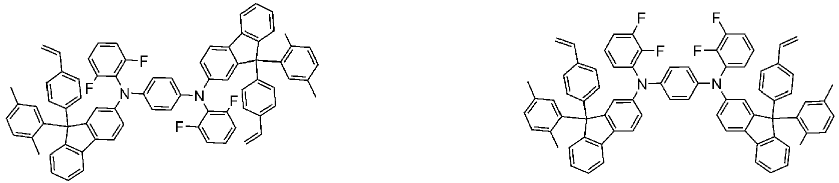

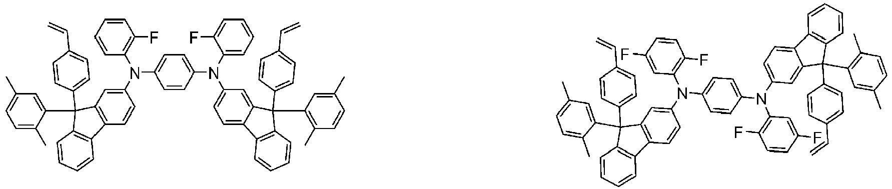

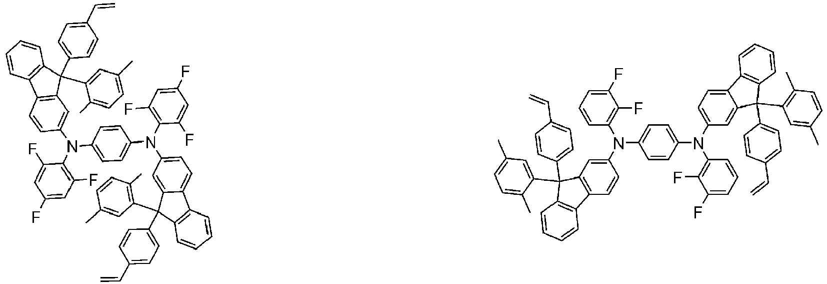

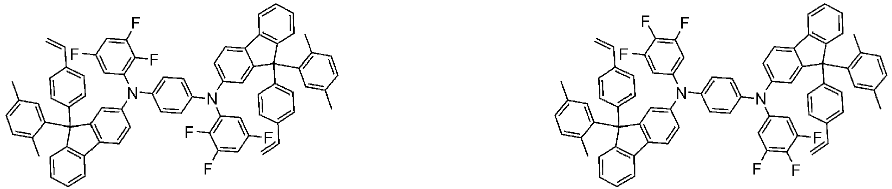

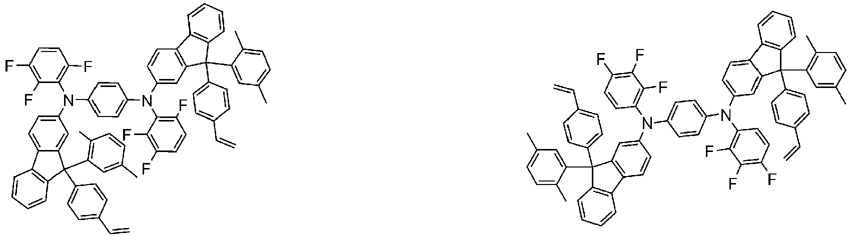

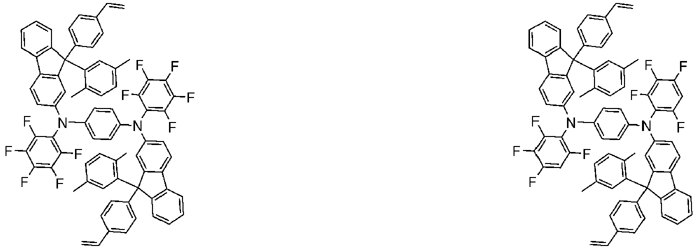

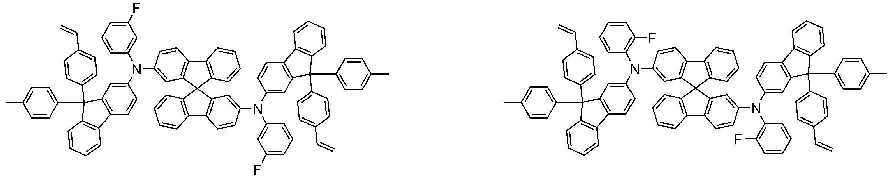

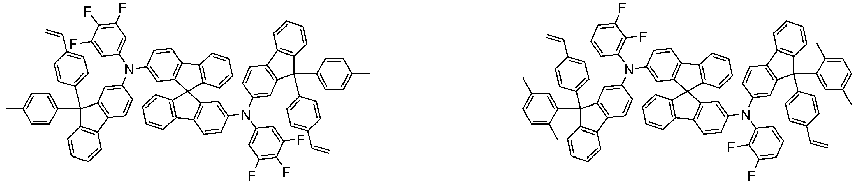

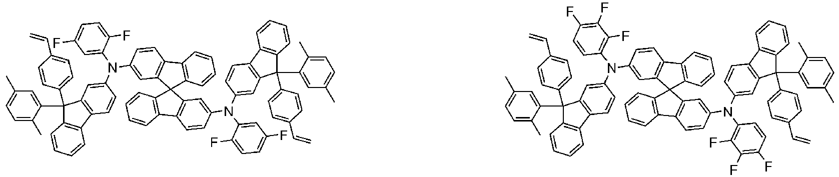

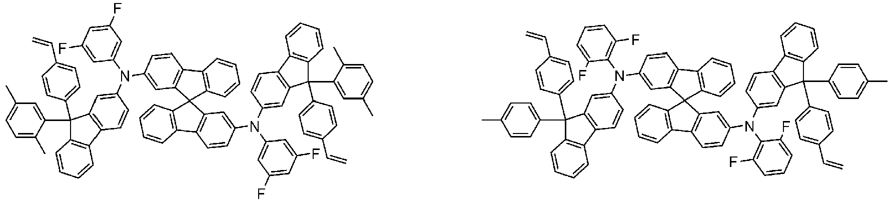

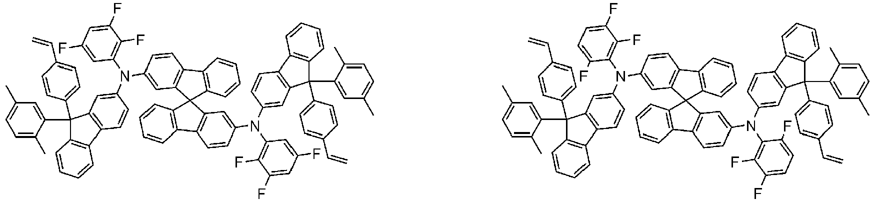

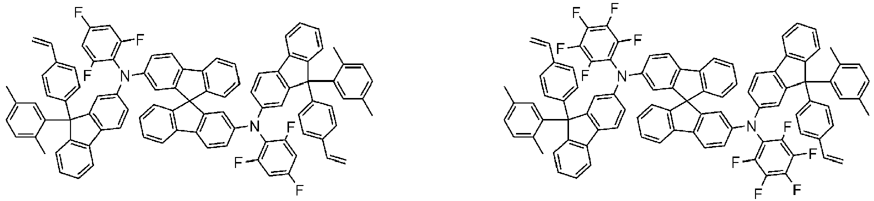

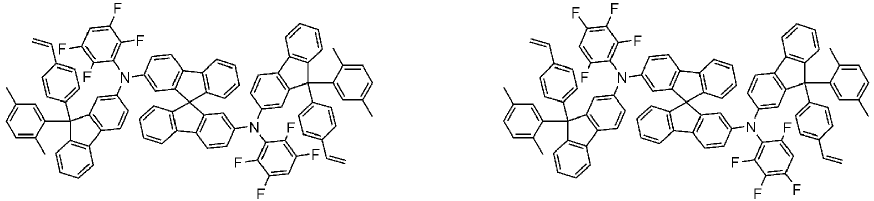

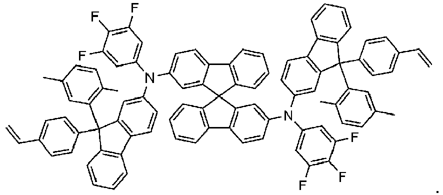

11. An organic light emitting device comprising
a first electrode;
a second electrode provided opposite the first electrode; and
one or more organic material layers provided between the first electrode and the second electrode, wherein one or more of the organic material layers include a cured product of the compound according to any one of claims 1 to 10.

## Patentansprüche

1. Verbindung, dargestellt durch die folgende chemische Formel 1: 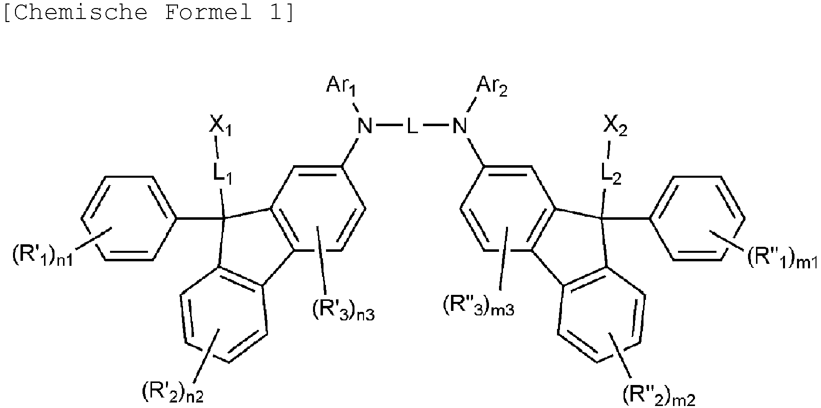 worin
L substituiertes oder unsubstituiertes C₆₋₆₀-Arylen oder substituiertes oder unsubstituiertes C₂₋₆₀-Heteroarylen, das irgendeines oder mehrere, aus N, O und S ausgewählte(s) Heteroatom(e) enthält, ist;
L₁ und L₂ jeweils unabhängig eine Einfachbindung oder Methylen sind;
X₁ und X₂ jeweils unabhängig eine fotohärtbare Gruppe oder eine warmhärtende Gruppe sind, unabhängig ausgewählt aus -L"-R", worin L" eine Einfachbindung, -O-, -S-,-CH₂-, -CH₂O-, -OCH₂- oder -CH₂OCH₂- ist und R" irgendeines der Folgenden ist: 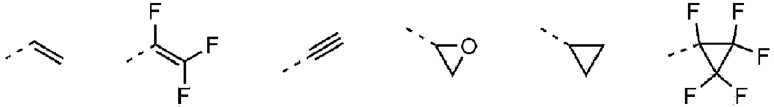 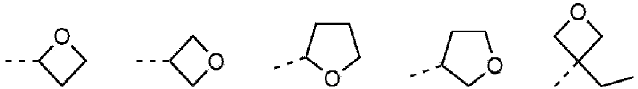 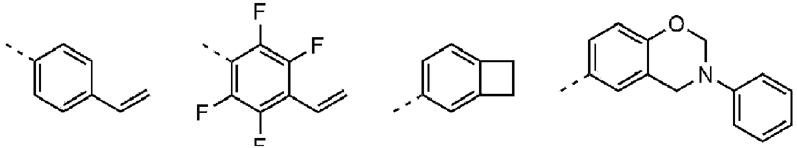
R'₁ bis R'₃ und R"₁ bis R"₃ jeweils unabhängig Wasserstoff, Deuterium, substituiertes oder unsubstituiertes C₁₋₆₀-Alkyl, substituiertes oder unsubstituiertes C₁₋₆₀-Alkoxy, substituiertes oder unsubstituiertes C₆₋₆₀-Aryl oder substituiertes oder unsubstituiertes C₂₋₆₀-Heteroaryl, enthaltend irgendeines oder mehrere Heteroatome, ausgewählt aus N, O und S, sind;
n1 bis n3 und m1 bis m3 jeweils unabhängig eine ganze Zahl von 0 bis 3 sind; und
Ar₁ und Ar₂ jeweils unabhängig eine Substituentengruppe sind, dargestellt durch die folgende chemische Formel 2: 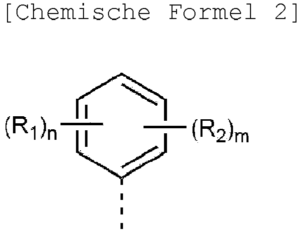
worin
jedes R₁ unabhängig ein Halogen ist;
jedes R₂ unabhängig Wasserstoff, Deuterium oder C₁₋₁₀-Alkyl ist; und
n eine ganze Zahl von 1 bis 5 ist und m 0 oder 1 ist, vorausgesetzt, dass m+n 5 oder kleiner ist.

2. Verbindung gemäß Anspruch 1, worin L Phenylen, Biphenyldiyl oder Spirobifluorendiyl ist.

3. Verbindung gemäß Anspruch 1, worin L irgendeines der Folgenden ist:

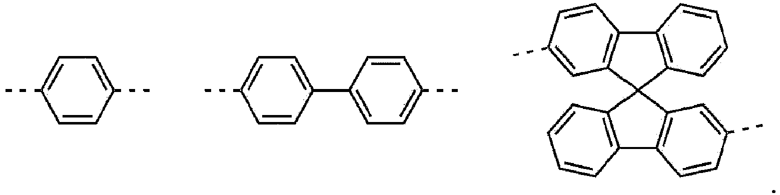

4. Verbindung gemäß Anspruch 1, worin R₁ Fluor ist.

5. Verbindung gemäß Anspruch 1, worin R₂ Wasserstoff, Deuterium oder Methyl ist.

6. Verbindung gemäß Anspruch 1, worin Ar₁ und Ar₂ jeweils unabhängig eine Substituentengruppe sind, dargestellt durch: 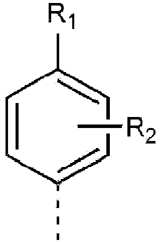 worin
R₁ C₁₋₁₀-Alkyl oder ein Halogen ist; und
R₂ Wasserstoff, Deuterium, C₁₋₁₀-Alkyl oder ein Halogen ist;
vorausgesetzt, dass zumindest eines von R₁ und R₂ ein Halogen ist.

7. Verbindung gemäß Anspruch 1, worin Ar₁ und Ar₂ jeweils unabhängig irgendeines der Folgenden sind:

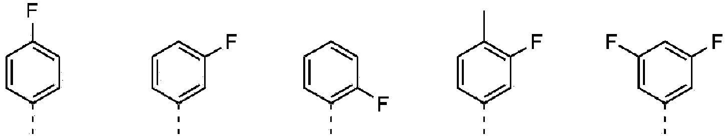

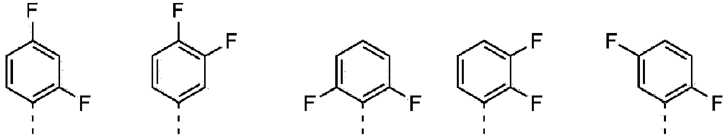

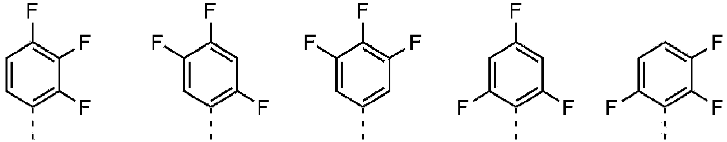

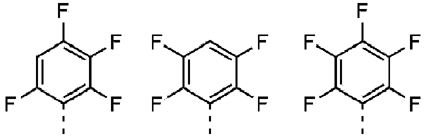

8. Verbindung gemäß Anspruch 1, worin
R'₁ und R"₁ jeweils unabhängig Wasserstoff oder Methyl sind; und
n1 und m1 jeweils unabhängig eine ganze Zahl von 0 bis 2 sind.

9. Verbindung gemäß Anspruch 1, worin R'₂, R'₃, R"₂ und R"₃ Wasserstoff sind.

10. Verbindung gemäß Anspruch 1, die irgendeine der Folgenden ist:

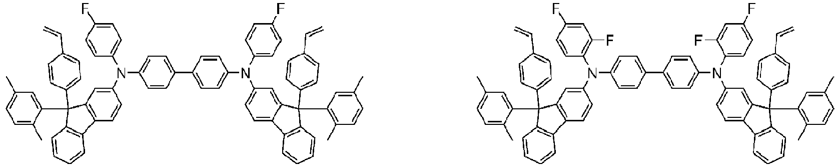

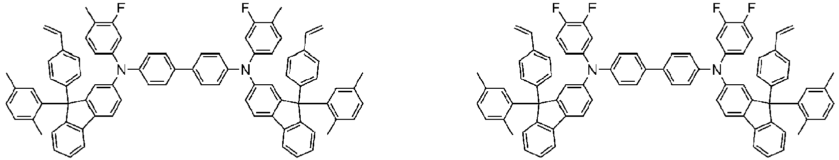

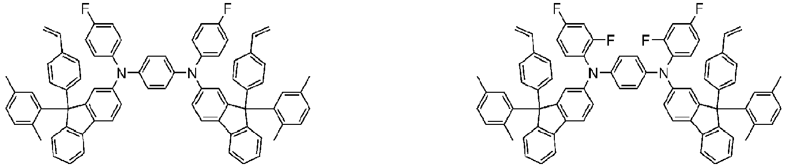

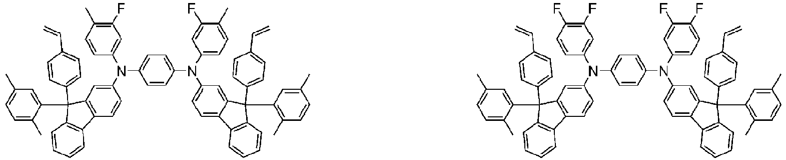

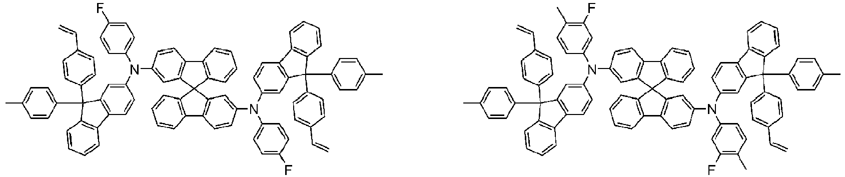

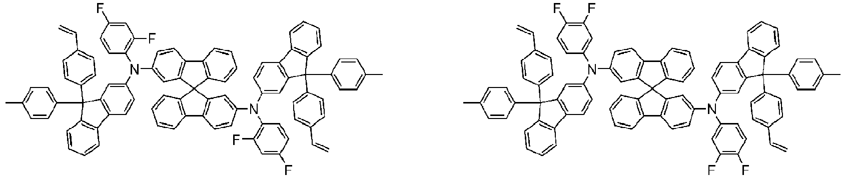

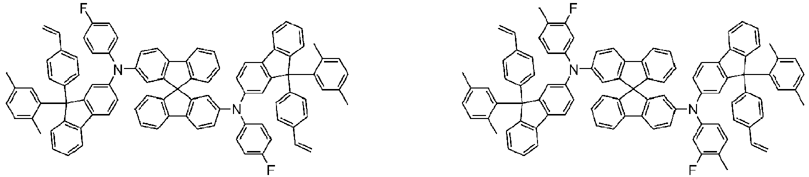

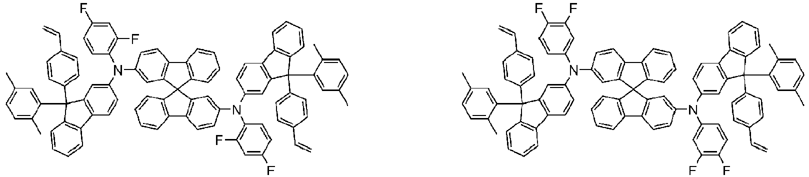

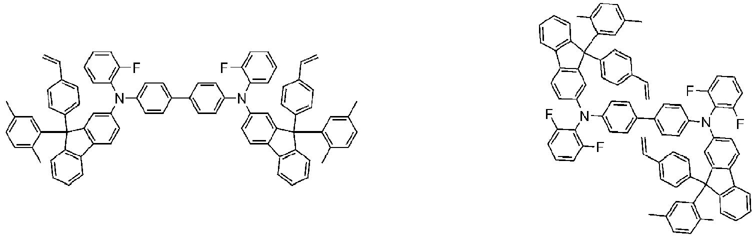

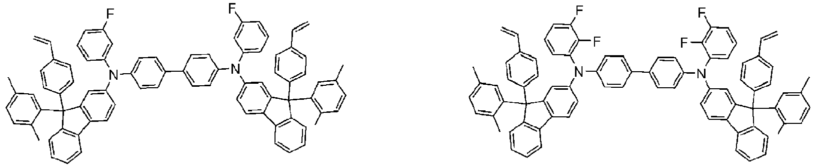

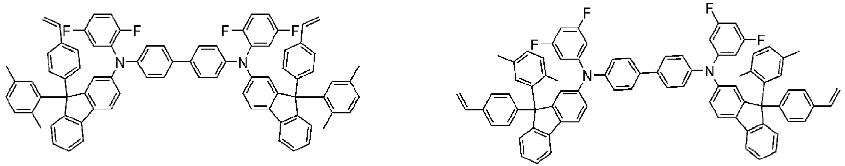

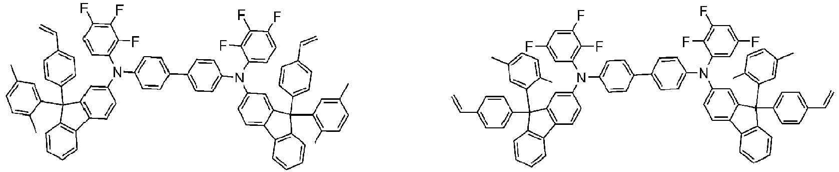

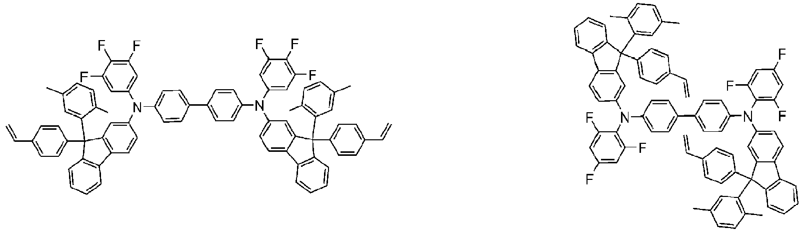

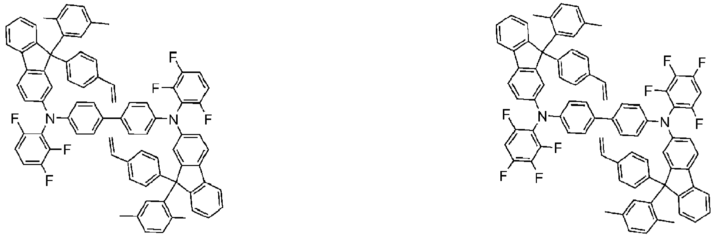

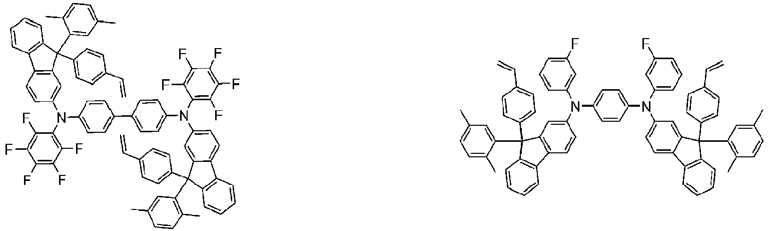

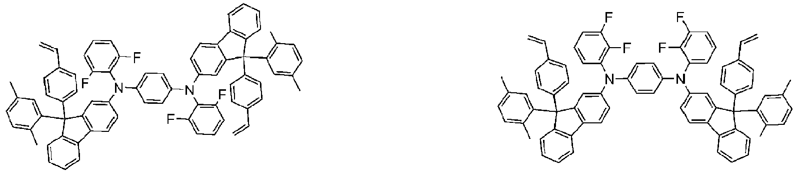

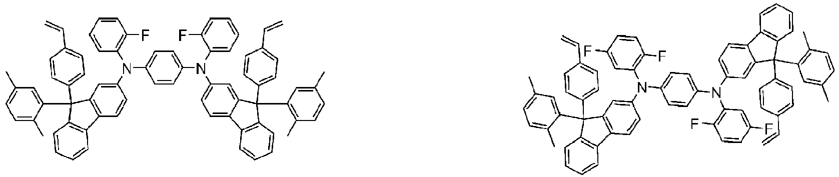

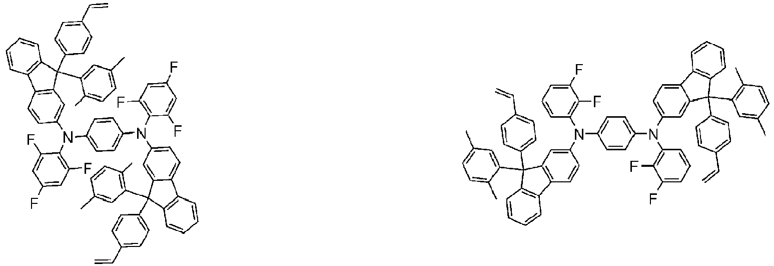

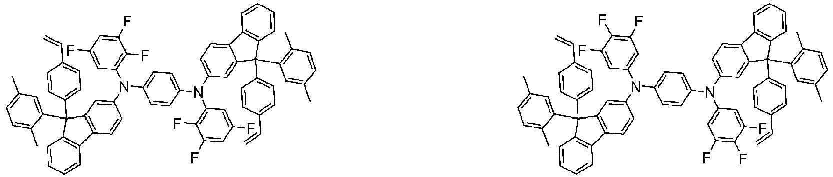

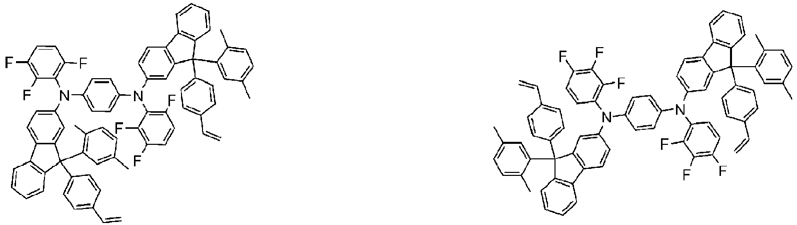

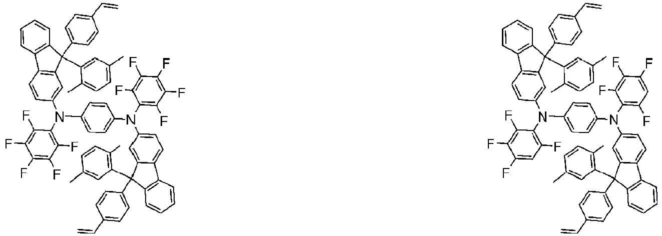

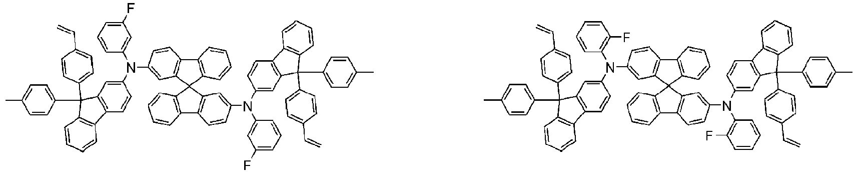

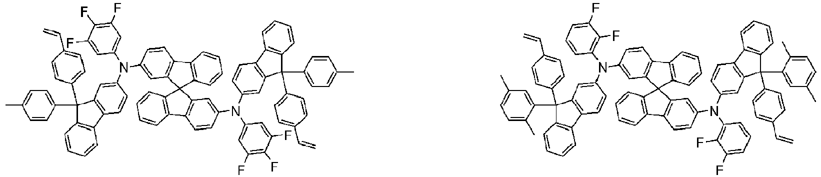

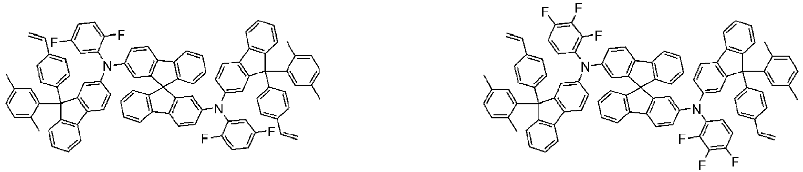

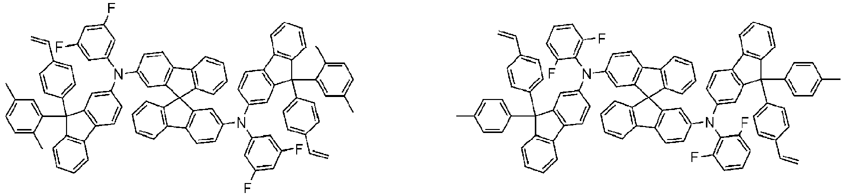

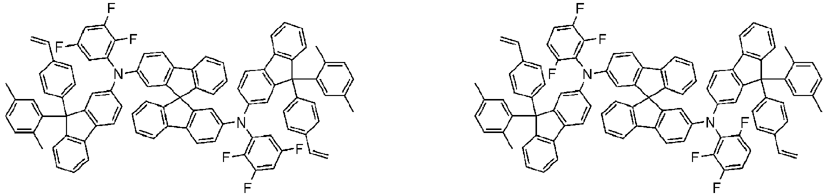

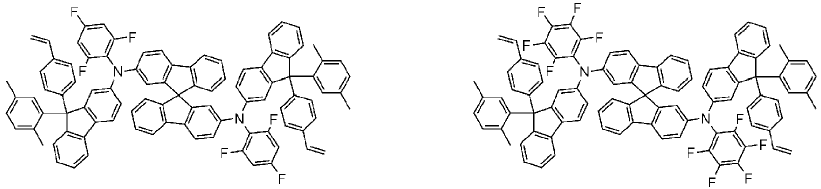

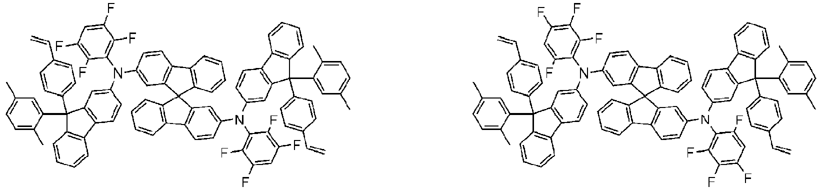

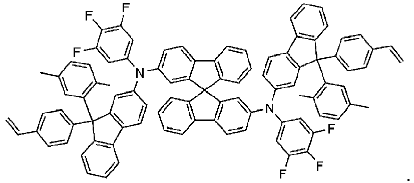

11. Organische lichtemittierende Vorrichtung, umfassend:
eine erste Elektrode;
eine zweite Elektrode, die gegenüberliegend zur ersten Elektrode vorgesehen ist; und
eine oder mehr organische Materialschicht(en), vorgesehen zwischen der ersten Elektrode und der zweiten Elektrode, worin eine oder mehr der organischen Materialschicht(en) ein gehärtetes Produkt der Verbindung gemäß irgendeinem der Ansprüche 1 bis 10 umfasst.

## Revendications

1. Composé représenté par la formule chimique 1 suivante : 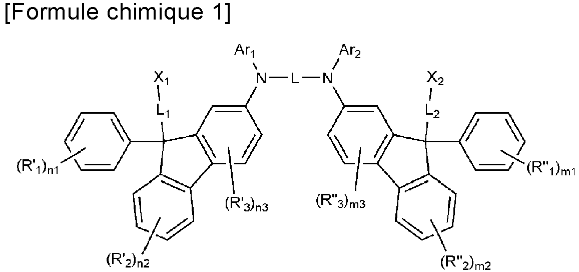 dans lequel
L est un arylène en C₆₋₆₀ substitué ou non substitué, ou hétéroarylène en C₂₋₆₀ substitué ou non substitué contenant un quelconque ou plusieurs hétéroatomes choisis parmi N, O et S ;
L₁ et L₂ sont chacun indépendamment une liaison simple ou du méthylène ;
X₁ et X₂ sont chacun indépendamment un groupe photodurcissable ou un groupe thermodurcissable représenté indépendamment par -L"-R", dans lequel L" est une liaison simple, -O-, -S-, -CH₂-, -CH₂O-, -OCH₂-, ou -CH₂OCH₂-, et R" est un élément quelconque parmi ce qui suit : 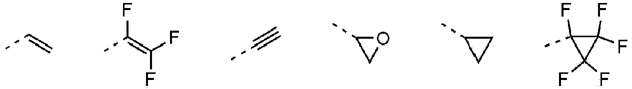 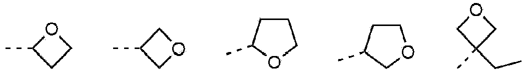 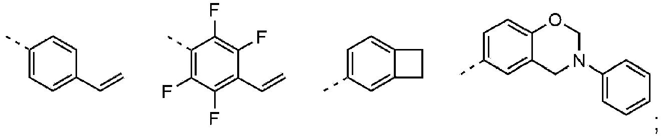
R'₁ à R'₃ et R"₁ à R"₃ sont chacun indépendamment de l'hydrogène, deutérium, alkyle en C₁₋₆₀ substitué ou non substitué, alcoxy en C₁₋₆₀ substitué ou non substitué, aryle en C₆₋₆₀ substitué ou non substitué, ou hétéroaryle en C₂₋₆₀ substitué ou non substitué contenant un quelconque ou plusieurs hétéroatomes choisis parmi N, O et S ;
n1 à n3 et m1 à m3 sont chacun indépendamment un nombre entier de 0 à 3 ; et
Ar₁ et Ar₂ sont chacun indépendamment un groupe substituant représenté par la formule chimique 2 : 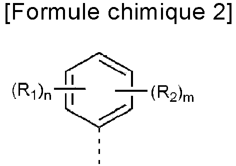 dans lequel
chaque R₁ est indépendamment un halogène ;
chaque R₂ est indépendamment de l'hydrogène, deutérium, ou alkyle en C₁₋₁₀ ; et
n est un nombre entier de 1 à 5, et m est 0 ou 1, à condition que n+m est 5 ou moins.

2. Composé selon la revendication 1, dans lequel L est du phénylène, biphényldiyle, ou spirobifluorènediyle.

3. Composé selon la revendication 1, dans lequel L est un élément quelconque parmi ce qui suit :

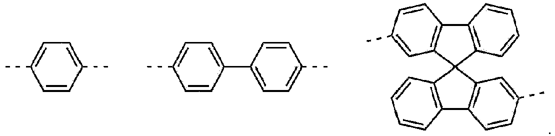

4. Composé selon la revendication 1, dans lequel R₁ est du fluoro.

5. Composé selon la revendication 1, dans lequel R₂ est de l'hydrogène, deutérium, ou méthyle.

6. Composé selon la revendication 1, dans lequel Ar₁ et Ar₂ sont chacun indépendamment un groupe substituant représenté par : 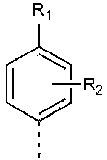 dans lequel
R₁ est un alkyle en C₁₋₁₀ ou un halogène ; et
R₂ est de l'hydrogène, deutérium, alkyle en C₁₋₁₀ ou un halogène ;
à la condition qu'au moins un élément parmi R₁ et R₂ soit un halogène.

7. Composé selon la revendication 1, dans lequel Ar₁ et Ar₂ sont chacun indépendamment un élément quelconque parmi ce qui suit :

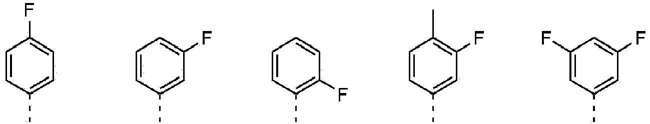

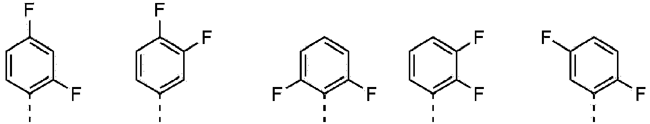

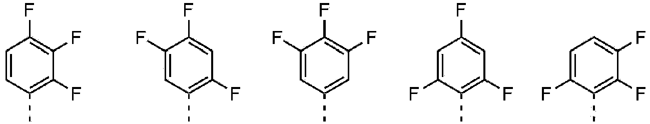

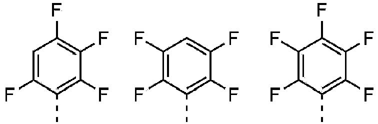

8. Composé selon la revendication 1, dans lequel R'₁ et R"₁ sont chacun indépendamment de l'hydrogène ou du méthyle ; et n1 et m1 sont chacun indépendamment un nombre entier de 0 à 2.

9. Composé selon la revendication 1, dans lequel R'₂, R'₃, R"₂ et R"₃ sont de l'hydrogène.

10. Composé selon la revendication 1, qui est un élément quelconque parmi ce qui suit :

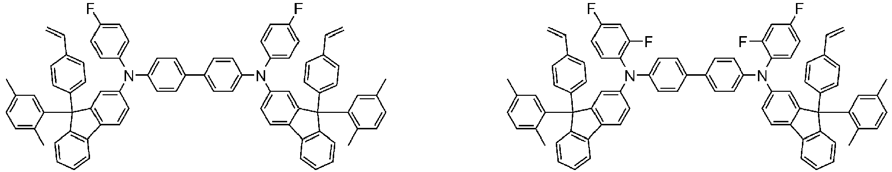

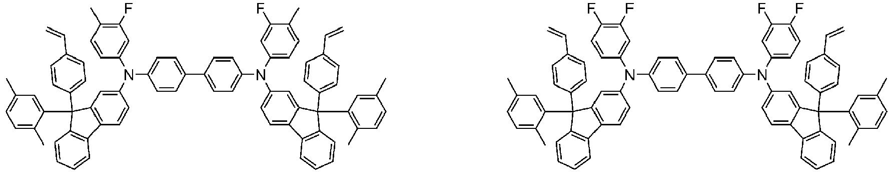

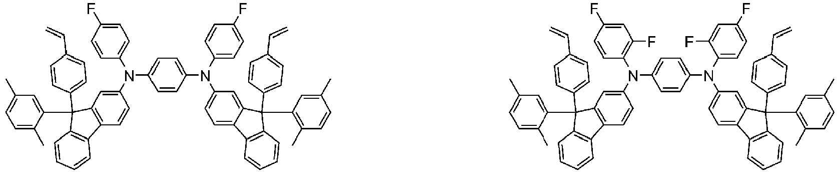

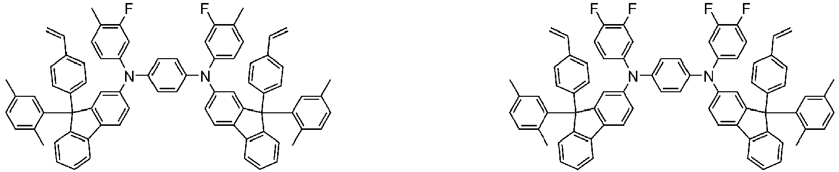

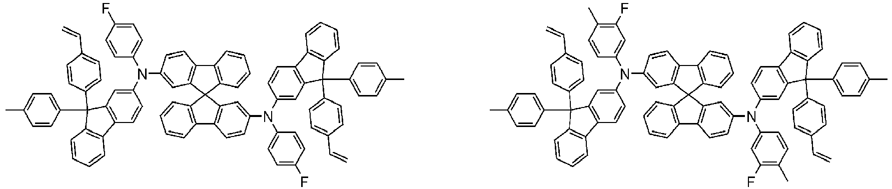

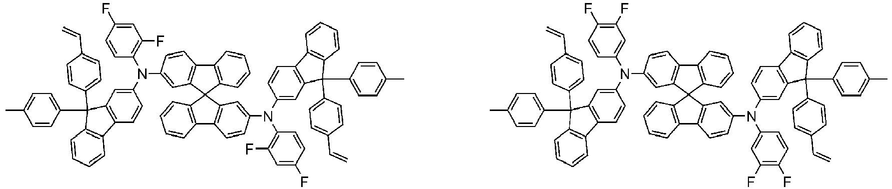

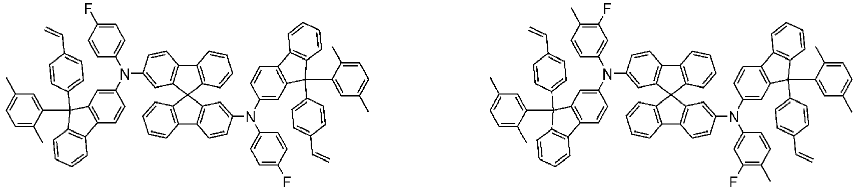

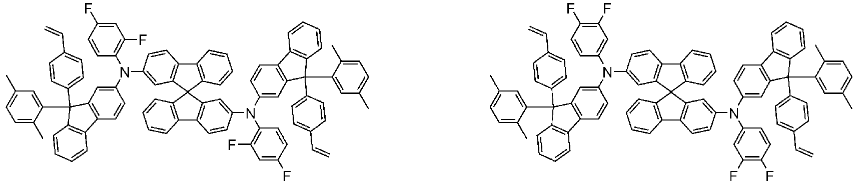

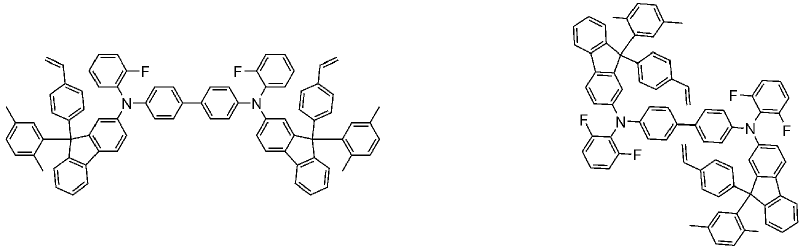

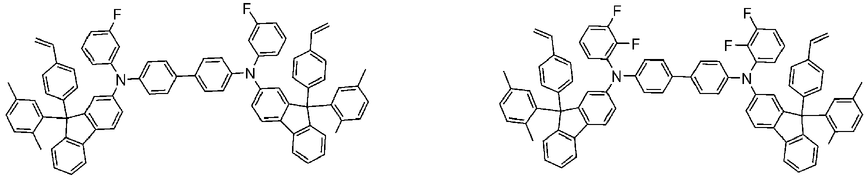

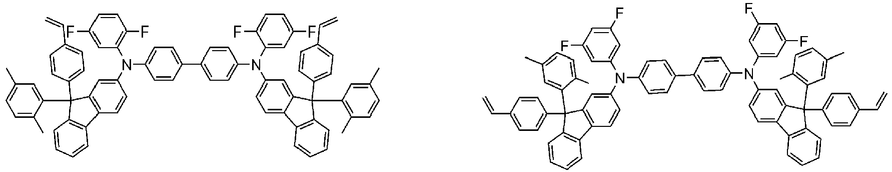

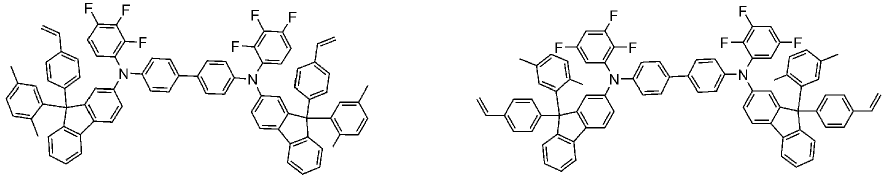

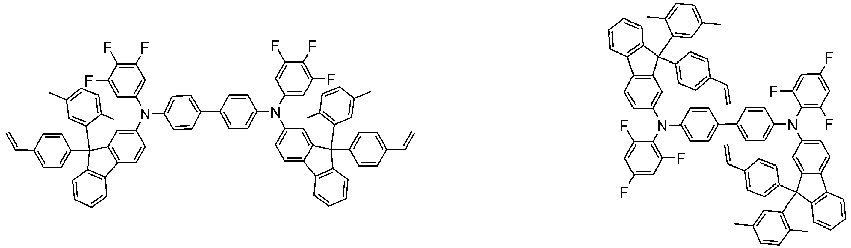

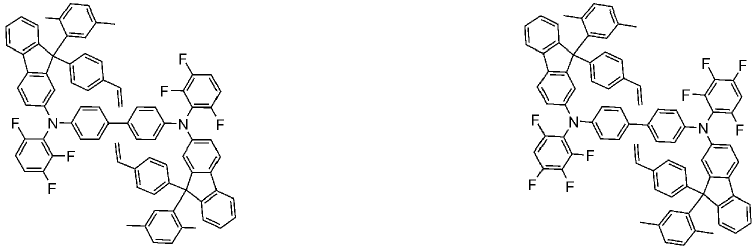

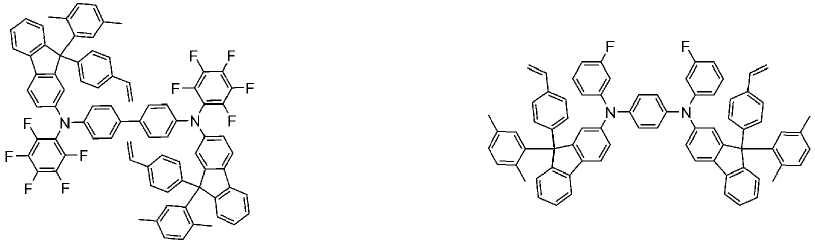

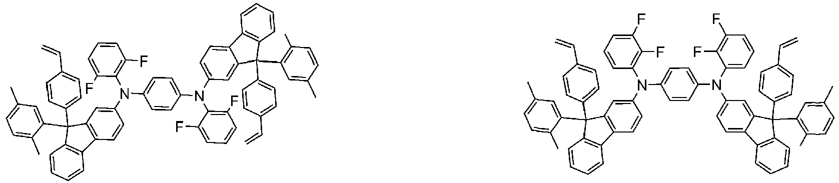

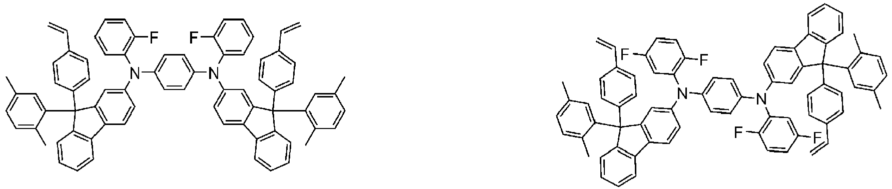

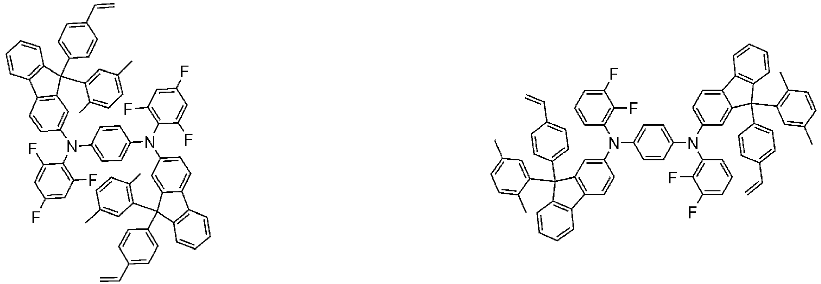

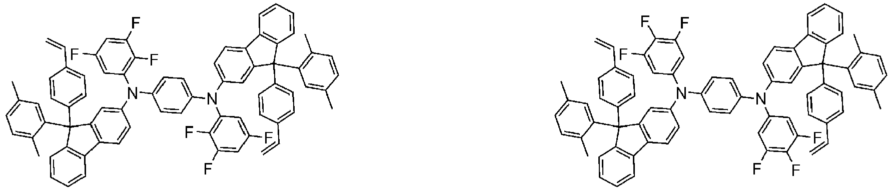

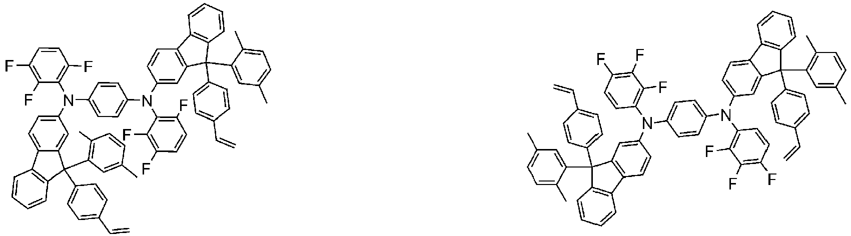

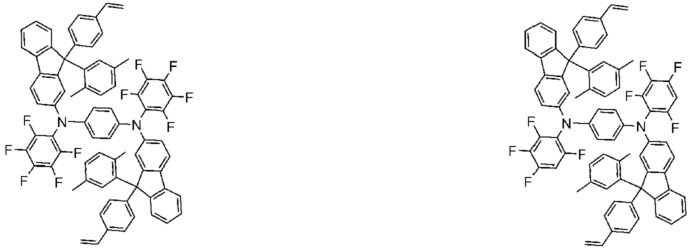

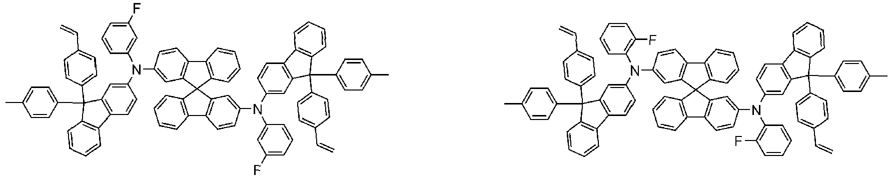

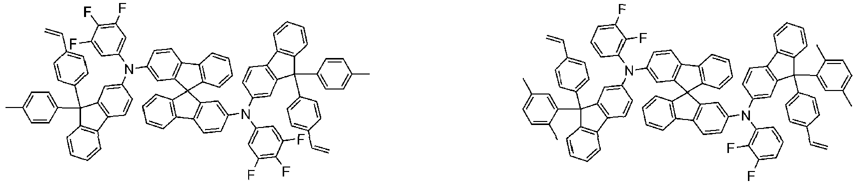

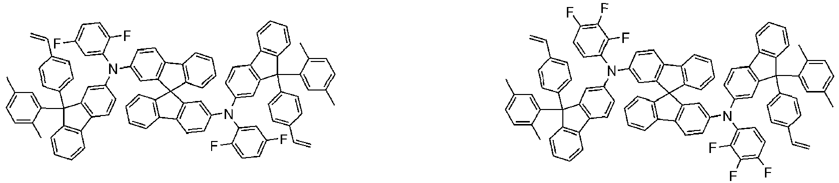

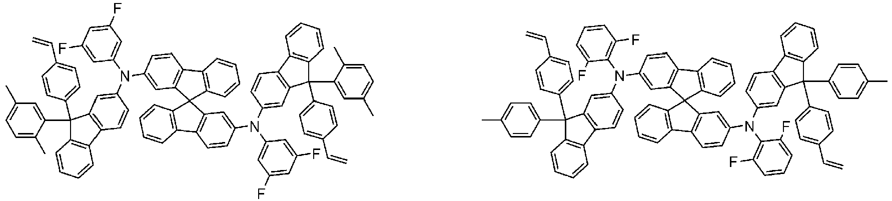

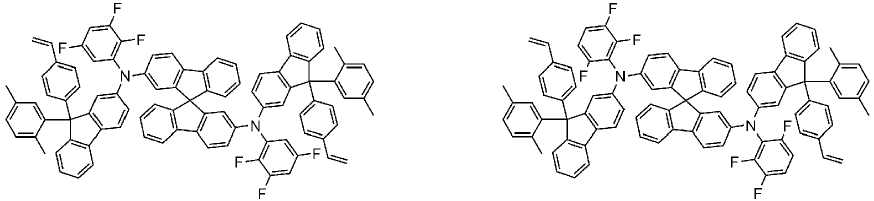

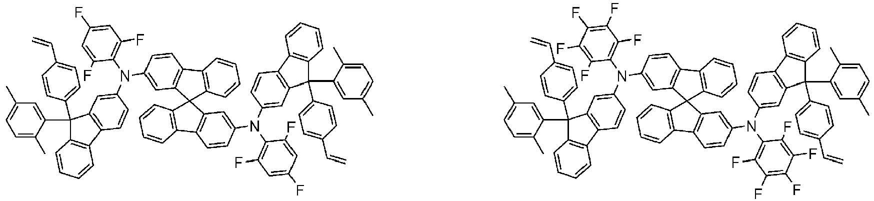

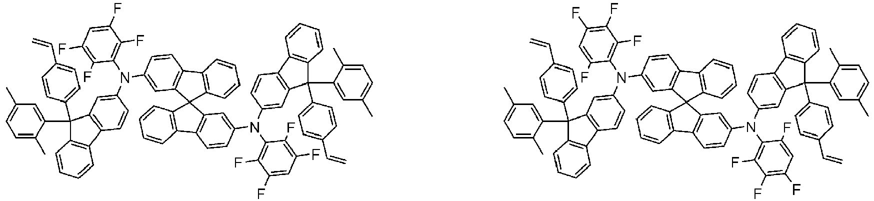

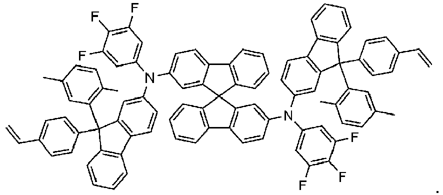

11. Dispositif électroluminescent organique comprenant
une première électrode ;
une seconde électrode prévue à l'opposé de la première électrode ; et
une ou plusieurs couches de matériau organique prévues entre la première électrode et la seconde électrode, dans lequel une ou plusieurs des couches de matériau organique incluent un produit durci du composé selon l'une quelconque des revendications 1 à 10.
